# EUROPÄISCHE PATENTSCHRIFT

(11) **EP 2 616 040 B1**
(45) Veröffentlichungstag und Bekanntmachung des Hinweises auf die Patenterteilung: **22.10.2014**
(21) Anmeldenummer: 11752111.2
(22) Anmeldetag: 18.08.2011
(51) Int. Cl.: A61K 8/49

(54) **2,2'-FUROIN-DERIVATE UND DEREN VERWENDUNG ZUR HAUTAUFHELLUNG**
2,2'-FUROIN DERIVATIVES AND THEIR USE FOR SKIN LIGHTENING
DÉRIVÉS 2,2'-FUROIN ET LEUR UTILISATION POUR ECLAIRCIR LA PEAU

(30) Priorität: 17.09.2010 DE 102010045890
(43) Veröffentlichungstag der Anmeldung: 24.07.2013
(73) Patentinhaber: Merck Patent GmbH, 64293 Darmstadt (DE)
(72) Erfinder: SCHEURICH, René, Peter, 64846 Gross-Zimmern (DE)
(86) Internationale Anmeldenummer: PCT/EP2011/004160
(87) Internationale Veröffentlichungsnummer: WO 2012/034629

(56) Entgegenhaltungen:
- WO-A1-2009/045709

## Beschreibung

Die Erfindung betrifft die Verwendung von Verbindungen der Formel (I) 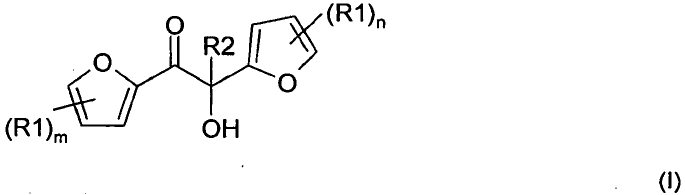 worin R1, R2, m und n die in den Ansprüchen angegebenen Bedeutungen aufweisen, und/oder ihre physiologisch unbedenklichen Salze, Tautomere, Stereoisomere und/oder Solvate, einschließlich deren Mischungen in allen Verhältnissen, zur Hemmung von Tyrosinase in-vitro sowie zur nicht-therapeutischen Aufhellung von Haut. Gegenstand der Erfindung sind auch die Verbindungen der Formel (I) zur Verwendung in der Prophylaxe, Therapie oder Verlaufskontrolle von Pigmentstörungen der Haut. Die Erfindung betrifft ferner Zubereitungen mit den Verbindungen der Formel (I) in Kombination mit mindestens einem weiteren Wirkstoff und ein Verfahren zum Herstellen der Zubereitungen durch Mischen der Verbindungen der Formel (I) mit einem für topische Anwendungen physiologisch unbedenklichen Träger.

Haut- und Haarfarbe sind von Gehalt, Größe und Typ des stickstoffhaltigen, dunklen Farbstoffs Melanin abhängig, das in den zur Melaninbildung befähigten Zellen (Melanozyten) produziert wird. Ausgehend von Tyrosin und mit Hilfe von verschiedenen Melanozyten-spezifischen Enzymen, wie z.B. Tyrosinase oder Tyrosinase-verwandten Proteinen, wird Melanin innerhalb der Melanozyten synthetisiert. Anschließend wird das Melanin in Form sogenannter Melanosomen zu den Keratinozyten transferiert. Obwohl das Melanin in der Haut ein geeigneter Schutz gegen UV-Strahlung ist, kann dunklere oder überpigmentierte Haut die Schönheit beeinflussen und zu ernsthaften ästhetischen Problemen führen. Hyperpigmentierte Hautpartien oder Läsionen enthalten Melasma (auch Chloasma genannt), d.h. unregelmäßig gestaltete gelblich-braune Flecken.

Generell unterscheidet man bei Pigmentflecken zwischen Sommersprossen (Epheliden), Altersflecken (Lentigines), sogenannten Alterswarzen (Verrucae seborrhoicea) und einer Hyperpigmentierung (z.B. Chloasma oder Melasma). Zu Sommersprossen neigen vor allem Menschen mit sehr heller Haut und rötlichen Haaren. Hyperpigmentierung (Chloasma) findet man hingegen häufig bei jenen Frauen, die regelmäßig ihrem Körper Östrogene zuführen. Sehr häufig spielt die Sonne eine wichtige Rolle. Vorbeugen kann man vor allem durch regelmäßigen Sonnenschutz mit einem hohen Lichtschutzfaktor. Um unschöne Pigmentflecken zu entfernen, bieten sich verschiedene Möglichkeiten wie Laser, Dermabrasio oder andere elektrochirurgische Verfahren sowie sogenannte Bleichcremes an. Letztere Alternative hat den Vorteil, dass sie für den Patienten wesentlich kostengünstiger als die elektrochirurgischen Verfahren ist. Außerdem ist die Anwendung einfacher und angenehmer.

WO 2009/045709 offenbart furan-substituierte Organosiloxane, die als Mittel zur Hautaufhellung dienen.

Eine große Zahl von Verbindungen mit hautaufhellender Wirkung zur Behandlung von Pigmentflecken ist auf dem Markt verfügbar. Unter anderem sind dies Verbindungen, wie z.B. Kojisäure, Arbutin, Aloesin, Niacinamid, Vitamin C oder Rucinol, die die Melaninproduktion in der Haut unterbinden. Dies kann unter Nutzung verschiedener Mechanismen geschehen. Meistens verzögern hautaufhellende Substanzen jedoch die Umwandlung von Tyrosin in Melanin durch Btockade des Enzyms Tyrosinase. Diese Verbindungen haben jedoch eine Reihe von Nachteilen, wie z.B. geringe Depigmentierungs-Effizienz, Nebenwirkungen wie Hautirritationen oder Hautexfoliation (Hautabschälung), Zellschädigungen, geringe Hautdurchdringung oder geringe Haltbarkeit bzw. Stabilität der Formulierungen. Daher ist ein Bedürfnis nach neuen sicheren Hautaufhellern mit höherer Effektivität und guten Formulierungseigenschaften vorhanden.

Der Erfindung liegt die Aufgabe zugrunde, die im Stand der Technik aufgezeigten Nachteile zu überwinden und wirksame Verbindungen zu entwickeln, die eine effektive Fähigkeit zur Hautaufhellung besitzen - mit dem Ziel, die kosmetische oder therapeutische Wirksamkeit bei gleichzeitiger Verringerung der Nebenwirkungen zu verbessern.

Die Aufgabe der Erfindung wird gemäß den unabhängigen Ansprüchen gelöst. Die Unteransprüche beinhalten bevorzugte Ausführungsformen. Erfindungsgemäß werden die Verbindungen der Formel (I) 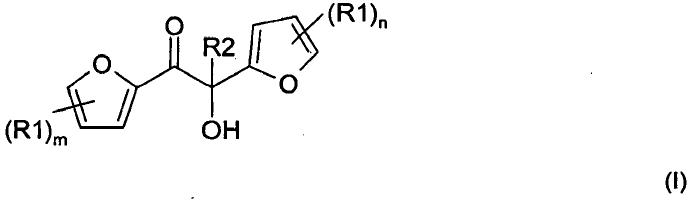 worin
- R1: A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
- R2: H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
- Y: H oder Alk,
- A: unverzweigtes oder verzweigtes Alkyl mit 1-18 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -CΞC- Gruppe ersetzt sein können,
- Alk: unverzweigtes oder verzweigtes Alkyl mit 1-12 C-Atomen,
- Cyc: zyklisches Alkyl mit 3-10 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -CΞC- Gruppe ersetzt sein können,
- Ar: einen ein-, zwei- oder dreikernigen aromatischen Carbozyklus mit 3 bis 20 C-Atomen, der unsubstituiert oder ein-, zwei- oder dreifach durch F, A, -(CY₂)ₚ-OY, -(CY₂)ₚ-NYY, -(CY₂)ₚ-COOY, -(CY₂)ₚ-CO-NYY oder -(CY₂)ₚ-NY-COY substituiert sein kann, und
- m, n, p: unabhängig voneinander 0, 1, 2 oder 3 bedeuten,
und/oder ihre physiologisch unbedenklichen Salze, Tautomere, Stereoisomere und/oder Solvate, einschließlich deren Mischungen in allen Verhältnissen,
zur Aufhellung der Haut verwendet. Es versteht sich dabei im Sinne der Erfindung, dass die Aufhellung der Haut eine nicht-therapeutische Verwendung darstellt.

Ebenso erfindungsgemäß werden die Verbindungen der Formel (I), wie zuvor beschrieben, und/oder ihre physiologisch unbedenklichen Salze, Tautomere, Stereoisomere und/oder Solvate, einschließlich deren Mischungen in allen Verhältnissen, zur Hemmung von Tyrosinase in-vitro verwendet.

In einer weiteren Ausgestaltung der Erfindung werden die Verbindungen der Formel (I), wie zuvor beschrieben, und/oder ihre physiologisch unbedenklichen Salze, Tautomere, Stereoisomere und/oder Solvate, einschließlich deren Mischungen in allen Verhältnissen, zur Prophylaxe, Therapie und/oder Verlaufskontrolle von Pigmentstörungen der Haut verwendet.

Überraschenderweise hat sich gezeigt, dass die Verbindungen der Formel (I) mit inhibierenden Eigenschaften auf die Tyrosinase versehen sind. Die Verbindungen der Formel (I) und insbesondere der Teilformeln (IA), (IB) und (IC) sind durch ihre Kernstruktur des 2,2'-Furoins, dem die Substituenten R1 und R2 anhaften, derart ausgestaltet, dass eine potente und selektive Hemmung der Tyrosinase erfolgt. Die Verwendung der Verbindungen mit der Formel (I) eröffnet damit völlig neue Möglichkeiten hinsichtlich der hautaufhellenden Wirkung. Bemerkenswerterweise spielen die Verbindungen der Formel (I) eine Rolle in der Behandlung von Pigmentflecken aller Art, indem sie die Synthese von Melanin unterbinden und die Melanin-Überproduktion verhindern. Die Rolle der Verbindungen der Formel (I) kann dabei sowohl nicht-therapeutischer als auch therapeutischer Natur sein.

Es ist bisher lediglich aus WO 1996/09807 bekannt, dass 2-Hydroxy-1-ethanon-Derivate zum Färben von keratinhaltigen Fasern, wie z.B. menschlichen Haaren, geeignet sind. Die Derivate adressieren Färbungen im Orange-, Braun-, Rotbraun-, Blauschwarz- und Schwarzbereich, insbesondere bei gemeinsamer Verabreichung mit Verbindungen mit primärer und sekundärer Aminogruppe, stickstoffhaltigen Heterozyklen oder aromatischen Hydroxyverbindungen. Im Gegensatz dazu enthüllt die vorliegende Erfindung, dass gerade durch Verwendung der Verbindungen der Formel (I) und/oder ihrer physiologisch unbedenklichen Salze, Tautomere, Stereoisomere und/oder Solvate, einschließlich deren Mischungen in allen Verhältnissen, die Haut exzellent aufgehellt wird, speziell die menschliche Haut. Bevorzugt im Sinne der Erfindung ist die Verwendung von mindestens einer Verbindung der Formel (I), wie hierin inklusive Ausführungsformen beschrieben, zur kosmetischen Aufhellung der Haut, besonders bevorzugt zur kosmetischen Aufhellung der menschlichen Haut. Insofern können Pigmentflecken kosmetisch, d.h. nicht-therapeutisch behandelt werden, wobei die Pigmentflecken insbesondere Hyperpigmentierung, Sommersprossen, Altersflecken und/oder Sonnenflecken umfassen. Ebenso erfindungsgemäß können die beschriebenen Verbindungen der Formel (I) insbesondere im Fall der Hautalterung, wie sie z.B. umweltbedingt hervorgerufen sein kann, kosmetisch verwendet werden.

Die Verbindungen der Formel (I) und ihre Salze besitzen folglich bei guter Verträglichkeit gleichzeitig wertvolle kosmetische und/oder pharmakologische Eigenschaften, indem die Aufhellung der Haut mit einem relativen Melaningehalt von kleiner als 90 % einhergeht, vorzugsweise kleiner als 80 %, besonders bevorzugt kleiner als 70 %.

Insbesondere sind die beschriebenen Verbindungen der Formel (I) Tyrosinase-Inhibitoren und zeigen aufgrund dieser Eigenschaft die gewünschte Aktivität als Hautaufheller. Ein Gegenstand der Erfindung betrifft also die Verwendung von mindestens einer Verbindung der Formel (I) bzw. Teilformeln davon und/oder ihrer physiologisch unbedenklichen Salze, Tautomere, Stereoisomere und/oder Solvate, einschließlich deren Mischungen in allen Verhältnissen, zur Hemmung von Tyrosinase in-vitro. Der Begriff "Hemmung" bezieht sich auf jede Verringerung der Aktivität, die auf der Wirkung der spezifischen erfindungsgemäßen Verbindungen basiert, indem letztere in der Lage sind, mit dem Zielmolekül derart zu interagieren, dass eine Erkennung, Bindung und Blockierung ermöglicht wird. Die Verbindungen zeichnen sich durch hohe Affinität zur Tyrosinase aus, wodurch eine verlässliche Bindung und vorzugsweise vollständige Blockade der Oxidase-Aktivität sichergestellt wird. Die Verbindungen sind besonders bevorzugt mono-spezifisch, um eine ausschließliche und unmittelbare Erkennung der ausgewählten Oxidase zu garantieren. Der Begriff der "Erkennung" bezieht sich hierbei auf jede Art der Wechselwirkung zwischen der Verbindung und den genannten Zielmolekülen, insbesondere kovalente oder nichtkovalente Bindungen, wie z.B. eine kovalente Bindung, hydrophobe/ hydrophile Wechselwirkungen, van der Waals'sche Kräfte, Ionenbeziehung, Wasserstoffbrücken oder Ligand-Rezeptor-Wechselwirkungen.

Die Verwendung der Verbindungen der Formel (I) zeigt eine vorteilhafte biologische Aktivität, die in den hierin beschriebenen Tests nachweisbar ist, wie z.B. auf Enzymen basierenden Assays. Die Messung der Oxidase-Aktivität ist eine dem Fachmann wohlbekannte Technik.

Die vorgenannte Verwendung der Verbindungen kann in in-vitro Modellen geschehen. Die Suszeptibilität einer bestimmten Zelle gegenüber der Behandlung mit den Verbindungen der Formel (I) kann durch Testen in-vitro bestimmt werden. Typischerweise wird eine Kultur der Zelle mit einer erfindungsgemäßen Verbindung bei verschiedenen Konzentrationen für eine Zeitdauer inkubiert, die ausreicht, um den aktiven Mitteln zu ermöglichen, die Synthese von Melanin zu inhibieren, gewöhnlich zwischen ungefähr einer Stunde und einer Woche. Zum Testen in-vitro können kultivierte Zellen aus einer Biopsieprobe verwendet werden. Die Menge des nach der Behandlung zurückbleibenden Melanins in den Zellen wird dann bestimmt. Die Verwendung in-vitro erfolgt insbesondere an Proben von Säugerspezies, die an Pigmentstörungen der Haut leiden. Der Wirt oder Patient kann jeglicher Säugerspezies angehören, z. B. einer Primatenspezies, insbesondere Menschen, aber auch Nagetieren (einschließlich Mäusen, Ratten und Hamstern), Kaninchen, Pferden, Rindern, Hunden, Katzen usw. Tiermodelle sind für experimentelle Untersuchungen von Interesse, wobei sie ein Modell zur Behandlung einer Krankheit oder kosmetischen Anomalie des Menschen zur Verfügung stellen.

Das Testen mehrerer spezifischer Verbindungen ermöglicht die Auswahl des Wirkstoffs, der am besten für die Behandlung des Patienten geeignet erscheint. Die in-vivo Dosis der gewählten Verbindung wird vorteilhaft auf die Suszeptibilität der Tyrosinase und/oder Schwere der Pigmentstörung des Patienten mit Blick auf die in-vitro Daten abgestimmt, wodurch die therapeutische Wirksamkeit merklich erhöht ist. Die Dosis variiert abhängig von der verwendeten spezifischen Verbindung, der spezifischen Erkrankung, dem Patientenstatus usw. Typischerweise ist eine kosmetische Dosis ausreichend, um die unerwünschte Melaninmenge im Zielgewebe erheblich zu vermindern, während die Lebensqualität des Patienten aufrechterhalten und schließlich verbessert wird. Die nachfolgende Lehre der Erfindung und deren Ausführungsformen betreffend die Verbindungen der Formel (I) zur Verwendung zur Prophylaxe, Therapie und/oder Verlaufskontrolle ist gültig und ohne Einschränkungen auf die Verwendung der Verbindungen zur Hemmung der Tyrosinase-Aktivität in-vitro anwendbar, sofern es sinnvoll erscheint.

Die Verwendung wird im Allgemeinen fortgesetzt, bis eine erhebliche Verringerung der Tyrosinase-Aktivität und Melaninproduktion vorliegt, z.B. mindestens ca. 10 % Verminderung des Melaningehalts und kann fortgesetzt werden, bis im Wesentlichen keine unerwünschte Überproduktion von Melanin mehr im Körper nachgewiesen wird. Es versteht sich dabei, dass eine Hautaufhellung eine wiederholte oder andauernde Behandlung darstellt, da nach Absetzten der Präparate der Formel (I) die normale Melaninsyntheserate wieder aufgenommen wird. In derartigen Tests zeigen und bewirken die erfindungsgemäßen Verbindungen einen inhibierenden Effekt, der gewöhnlich durch IC₅₀-Werte in einem geeigneten Bereich, bevorzugt im mikromolaren Bereich und bevorzugter im nanomolaren Bereich dokumentiert wird. Die Tyrosinase wird insbesondere zu 50 % gehemmt, wenn die Konzentration der Verbindungen kleiner als 1 µM ist, vorzugsweise kleiner als 0,5 µM, besonders bevorzugt kleiner als 0,1 µM. Diese Konzentration wird als IC₅₀-Wert bezeichnet.

Erfindungsgemäß sind Verbindungen der Formel (I) und/oder ihre physiologisch unbedenklichen Salze, Tautomere, Stereoisomere und/oder Solvate, einschließlich deren Mischungen in allen Verhältnissen, zur Verwendung in der Prophylaxe, Therapie und/oder Verlaufskontrolle von Erkrankungen geeignet, die durch Aktivität der Tyrosinase hervorgerufen, vermittelt und/oder verbreitet werden. Zur Identifizierung eines entsprechenden Signalübertragungswegs und zum Nachweis von Wechselwirkungen zwischen verschiedenen Signalübertragungswegen sind geeignete Modelle oder Modellsysteme entwickelt worden, z.B. Zellkulturmodelle (Khwaja et al. (1997) EMBO 16: 2783) und Modelle transgener Tiere (White et al. (2001) Oncogene 20: 7064). Zur Bestimmung bestimmter Stufen in der Signalübertragungskaskade können wechselwirkende Verbindungen genutzt werden, um das Signal zu modulieren (Stephens et al. (2000) Biochemical J 351: 95). Darüber hinaus können die erfindungsgemäßen Verbindungen auch als Reagenzien zur Testung oxidaseabhängiger Signalübertragungswege in Tieren und/oder Zellkulturmodellen oder in den in dieser Anmeldung genannten klinischen Erkrankungen verwendet werden. Wie hierin besprochen, sind diese Signalwege für verschiedene Erkrankungen relevant. Dementsprechend sind die erfindungsgemäßen Verbindungen nützlich bei der Prophylaxe, Therapie und/oder Verlaufskontrolle von Erkrankungen, die von Signalwegen mit Beteiligung der Tyrosinase abhängig sind.

Erfindungsgemäß sind Verbindungen der Formel (I) bzw. deren Teilformeln und/oder ihre physiologisch unbedenklichen Salze, Tautomere, Stereoisomere und/oder Solvate, einschließlich deren Mischungen in allen Verhältnissen, zur Verwendung in der Prophylaxe, Therapie und/oder Verlaufskontrolle von Pigmentstörungen geeignet, die aus der Gruppe von Hyperpigmentierung, Sommersprossen, Altersflecken und Sonnenflecken ausgewählt sind.

Eine weitere Ausgestaltung der vorliegenden Erfindung betrifft die Verwendung der Verbindungen der Formel (I) und/oder ihre physiologisch unbedenklichen Salze, Tautomere, Stereoisomere und/oder Solvate, einschließlich deren Mischungen in allen Verhältnissen, in Kombination mit mindestens einem weiteren Wirkstoff, der bevorzugt aus der Gruppe von Antioxidantien, Vitaminen, UV-Filtern, hautaufhellenden Wirkstoffen, Selbstbräunungssubstanzen, anti-inflammatorischen Agenzien, antimikrobiellen Wirkstoffen, hautfeuchteverbessernden Wirkstoffen, alterungshemmenden Wirkstoffen (Anti-Aging-Wirkstoffen) und Anti-Cellulite-Wirkstoffen ausgewählt ist.

Im Rahmen der Erfindung sind die Verbindungen der Formel (I) so definiert, dass man darunter auch pharmazeutisch oder kosmetisch verwendbare Derivate, Salze, Hydrate, Solvate, Vorstufen der Verbindungen, Tautomere und optisch aktive Formen (wie z.B. Stereoisomere, Diastereomere, Enantiomere, Racemate) versteht. Unter Solvaten der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate. Unter pharmazeutisch oder kosmetisch verwendbaren Derivaten versteht man z.B. die Salze der erfindungsgemäßen Verbindungen als auch sogenannte Vorstufen der Verbindungen. Unter Vorstufen versteht man z.B. mit Alkyl- oder Acylgruppen, Zuckern oder Oligopeptiden abgewandelte Verbindungen der Formel (I), die im Organismus rasch zu den wirksamen erfindungsgemäßen Verbindungen gespalten werden. Hierzu gehören auch bioabbaubare Polymerderivate der erfindungsgemäßen Verbindungen, wie dies z.B. in Int. J. Pharm. 115, 61-67 (1995) beschrieben ist. Jegliche Verbindung, die in-vivo in ein bioaktives Mittel, d.h. Verbindungen der Formel (I) umgewandelt werden kann, ist eine Vorstufe im Sinne dieser Erfindung. Jegliche biologisch aktive Verbindung, die aus der in-vivo Verstoffwechslung einer erfindungsgemäßen Verbindung resultiert, ist ein Metabolit im Sinne der vorliegenden Erfindung. Die Verbindungen der Formel (I) können ein oder mehrere chirale Zentren besitzen und daher in verschiedenen stereoisomeren Formen vorkommen. Die Formel (I) schließt alle diese Formen ein.

Gegenstand der Erfindung ist auch die Verwendung von Mischungen der Verbindungen der Formel (I), z.B. Gemische zweier Diastereomerer, z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Vor- und nachstehend haben die Radikale R1, R2, Y, A, Alk, Cyc und Ar sowie die Indizes m, n und p die bei der Formel (I) angegebenen Bedeutungen, sofern nicht ausdrücklich etwas anderes angegeben ist. Bei mehrfachem Auftreten einzelner Reste innerhalb einer Verbindung oder eines Radikals nehmen die Reste unabhängig voneinander die angegebenen Bedeutungen an, sofern nicht ausdrücklich etwas anderes angegeben ist. Beispielsweise sind die Reste YY im Radikal R1, in dem sie mehrfach vorkommen, identisch oder verschieden, aber vorzugsweise in jedem Fall unabhängig voneinander unter den vorstehend und/oder nachstehend angegebenen Bedeutungen ausgewählt (z.B. Methyl und/oder Ethyl), sofern nicht ausdrücklich etwas anderes angegeben ist. Es versteht sich beispielsweise ebenso, dass in der Schreibung (R1)ₘ der Index m die Häufigkeit der Substitution durch das Radikal R1 angibt, d.h. der Furanylrest kann bis zu 3 Radikale R1 in unterschiedlichen Positionen tragen (nicht jedoch eine Verkettung von bis zu 3 Radikalen in derselben Position), wobei die jeweiligen Reste R1 identisch oder verschieden, aber vorzugsweise in jedem Fall unabhängig voneinander unter den vorstehend und/oder nachstehend angegebenen Bedeutungen ausgewählt sind. Darüber hinaus sind die Reste R1 in der Formel (I), in der sie generisch zweifach vorkommen, identisch oder verschieden, aber vorzugsweise in jedem Fall unabhängig voneinander unter den vor-. stehend und/oder nachstehend angegebenen Bedeutungen ausgewählt (z.B. A und/oder F). Bei mehrfachem Auftreten von R1 kann das Radikal alternativ auch mit R1', R1", R1"', R1"", R'"" und R""" bezeichnet werden. Den vorliegend verwendeten Bezeichnungen zur Definition der Verbindungen liegen im Allgemeinen die Regeln der IUPAC-Organisation für chemische Verbindungen und insbesondere organische Verbindungen zugrunde. Die Bezeichnungen zur Erläuterung der o.g. Verbindungen der Erfindung haben immer die nachstehenden Bedeutungen, sofern die Beschreibung oder die Ansprüche nicht etwas anderes anzeigen.

Der Begriff "unsubstituiert" bedeutet, dass ein Radikal, eine Gruppe oder eine Rest keine Substituenten trägt. Der Begriff "substituiert" bedeutet, dass ein Radikal, eine Gruppe oder ein Rest einen oder mehrere Substituenten trägt.

"Alkyl" oder "A" bezeichnet im Sinne der Erfindung ein gesättigtes oder ungesättigtes Kohlenwasserstoffradikal, das unverzweigt (linear) oder verzweigt ist und vorzugsweise 1, 2, 3, 4, 5, 6, 7, 8, 9, 10, 11, 12, 13, 14, 15, 16, 17 oder 18 C-Atome besitzt, d.h. C₁₋₁₈-Alkanyl, C₂₋₁₈-Alkenyle und C₂₋₁₈-Alkynyle. Beispiele für Alkylradikale sind Methyl, Ethyl, Propyl, Isopropyl, 1,1-, 1,2- oder 2,2-Dimethylpropyl, 1-Ethylpropyl, 1-Ethyl-1-methylpropyl, 1-Ethyl-2-methylpropyl, 1,1,2-oder 1,2,2-Trimethylpropyl, Butyl, Isobutyl, sek.-Butyl, tert.-Butyl, 1-, 2- oder 3-Methylbutyl, 1,1-, 1,2-, 1,3-, 2,2-, 2,3- oder 3,3-Dimethylbutyl, 1- oder 2-Ethylbutyl, Pentyl, Isopentyl, Neopentyl, tert.-Pentyl, 1-, 2-, 3- oder 4-Methylpentyl, Hexyl, Dodecyl. Des Weiteren sind Reste, die sich aus Öl-, Plamitin- oder Stearinsäure ableiten, üblich, insbesondere in der Kosmetik, da sie die Lipophilie wesentlich erhöhen und somit galenische und rheologische Eigenschaften gesteuert werden können. Alkenyle haben mindestens eine C-C Doppelbindung and Alkynyle mindestens eine C-C Dreifachbindung. Alkynyle können außerdem mindestens eine C-C Doppelbindung aufweisen. Beispiele für geeignete Alkenyle sind Allyl, Vinyl, Propenyl (-CH₂CH=CH₂; -CH=CH-CH₃; -C(=CH₂)-CH₃), 1-, 2- oder 3-Butenyl, Isobutenyl, 2-Methyl-1- oder 2-Butenyl, 3-Methyl-1-butenyl, 1,3-Butadienyl, 2-Methyl-1,3-butadienyl, 2,3-Dimethyl-1,3-butadienyl, 1-, 2-, 3- oder 4-Pentenyl and Hexenyl. Beispiele für geeignete Alkynyle sind Ethynyl, Propynyl (-CH₂-C≡CH; -C≡C-CH₃), 1-, 2- oder 3-Butynyl, Pentynyl, Hexynyl oder Pent-3-en-1-in-yl, insbesondere Propynyl. Auch Radikale, die bei denen mehrere CH₂-Gruppen durch -CH=CH- und -C≡C- ersetzt sein können, sind Teil der vorliegenden Erfindung. Enyne-Verbindungen sind synthetisch gut zugänglich und können in der Natur, z.B. durch Bergmanncyclisierung unter Aufbau zusätzlicher Ringsysteme weiter transformiert werden.

In einer bevorzugten Ausführungsform der Erfindung ist "A" unverzweigtes oder verzweigtes Alkyl mit 1, 2, 3, 4, 5, 6, 7, 8, 9, 10, 11, 12, 13, 14, 15, 16, 17 oder 18 C-Atomen, wobei unabhängig voneinander 1, 2 oder 3 H-Atome durch F und/oder unabhängig voneinander eine oder zwei benachbarte CH₂-Gruppen durch -O-, -N(Alk)-, eine -CH=CH- und/oder -CΞC- Gruppe ersetzt sein können. Besonders bevorzugt als "A" ist unverzweigtes oder verzweigtes Alkyl mit 1, 2, 3, 4, 5, 6, 7 oder 8 C-Atomen, wobei unabhängig voneinander 1, 2 oder 3 H-Atome durch F und/oder unabhängig voneinander eine oder zwei benachbarte CH₂-Gruppen durch -O-, -N(Alk)-, eine -CH=CH- und/oder -CΞC- Gruppe ersetzt sein können. Besonders bevorzugte Beispiele des substituierten "A" sind Hydroxyethyl, Hydroxypropyl, Alkoxyethyl (z.B. Methoxyethyl, Ethoxyethyl) und Alkoxypropyl (z.B. Methoxypropyl, Ethoxypropyl). Ganz besonders bevorzugt entspricht "A" der Bedeutung "Alk". Es versteht sich, dass die jeweiligen Bedeutungen von "A" unabhängig voneinander in den Radikalen einer erfindungsgemäßen Formel sind.

Der Begriff "Alk" bezeichnet im Sinne der Erfindung unverzweigtes oder verzweigtes Alkyl mit 1, 2, 3, 4, 5, 6, 7, 8, 9, 10, 11 oder 12 C-Atomen, wobei unabhängig voneinander 1, 2 oder 3 H-Atome durch F ersetzt sein können, vorzugsweise C₁₋₁₂-Alkyl, besonders bevorzugt C₁₋₄-Alkyl. Ein solches C₁₋₄-Alkyl ist ganz besonders bevorzugt Methyl, Ethyl, Propyl, Isopropyl, Butyl, Isobutyl, sek.-Butyl, tert.-Butyl, Fluormethyl, Difluormethyl, Trifluormethyl oder 1,1,1-Trifluorethyl, höchst bevorzugt Methyl, Ethyl oder Trifluormethyl. Es versteht sich, dass die jeweiligen Bedeutungen von "Alk" unabhängig voneinander in den Radikalen einer erfindungsgemäßen Formel sind.

"Cycloalkyl" oder "Cyc" bezeichnet im Sinne der Erfindung gesättigte und teilweise ungesättigte nicht-aromatische zyklische Kohlenwasserstoffgruppen mit 1 bis 3 Ringen, die 3 bis 20, vorzugsweise 3 bis 12, besonders bevorzugt 3 bis 10 C-Atome umfassen. Die Bindung an das Grundgerüst der Formel (I) kann über jedes Ringmitglied der Cycloalkylgruppe erfolgen. Beispiele für geeignetes Cycloalkyl sind Cyclopropyl, Cyclobutyl, Cyclopentyl, Cyclohexyl, Cycloheptyl, Cyclooctyl, Cyclodecyl, Cyclopentenyl, Cyclopentadienyl, Cyclohexenyl und Cyclooctadienyl sowie Adamantyl, Bicyclo[5.3.0]decapentaenyl (Azulenrest), 1,7,7-Trimethyl-bicyclo[2.2.1]heptan-2-on (Campherrest) oder 1-Methyl-4-prop-1-en-2-ylcyclohexenyl (Limonenrest).

In einer bevorzugten Ausführungsform der Erfindung ist "Cyc" zyklisches Alkyl mit 3, 4, 5, 6, 7, 8, 9 oder 10 C-Atomen, wobei unabhängig voneinander 1, 2 oder 3 H-Atome durch F und/oder unabhängig voneinander eine oder zwei benachbarte CH₂-Gruppen durch -O-, -N(Alk)-, eine -CH=CH- und/oder-CΞC- Gruppe ersetzt sein können. Besonders bevorzugt ist zyklisches Alkyl mit 3, 4, 5, 6, 7 oder 8 C-Atomen, wobei unabhängig voneinander 1, 2 oder 3 H-Atome durch F und/oder unabhängig voneinander eine oder zwei benachbarte CH₂-Gruppen durch -O-, -N(Alk)- oder eine -CH=CH- Gruppe ersetzt sein können. Ganz besonders bevorzugte Beispiele hierfür sind Cycloprenyl, Cyclobutenyl, Cyclopentenyl, Cyclopentadienyl, Cyclohexenyl, Cyclohexadienyl, Cyclooctadienyl oder Norbornyl.

Grundgerüst der Formel (I) ist jede generische oder nicht-generische Struktur, an die irgendein Radikal im Sinne der Erfindung, wie z.B. Cyc oder Ar, gebunden werden kann, um zu einer Verbindung der Formel (I) zu gelangen.

Der Begriff "Aryl", "Carboaryl" oder "Ar" bezeichnet im Sinne der Erfindung ein mono- oder polyzyklisches aromatisches Kohlenwasserstoffsystem mit 3, 4, 5, 6, 7, 8, 9, 10, 11, 12, 13, 14, 15, 16, 17, 18, 19 oder 20, vorzugsweise 5-14, besonders bevorzugt 5-10 C-Atomen, die gegebenenfalls substituiert sein können. Der Begriff "Aryl" schließt solche Systeme ein, in denen der aromatische Zyklus Teil eines bi- oder polyzyklischen gesättigten, teilweise ungesättigten und/oder aromatischen Systems ist, z.B. wenn der aromatische Zyklus an "Aryl", "Heteroaryl" oder "Heterocyclyl" über ein beliebiges Ringmitglied des Arylradikals fusioniert ist. Die Bindung an das Grundgerüst der Formel (I) kann über jedes Ringmitglied der Arylgruppe erfolgen. Beispiele für geeignetes "Aryl" sind Phenyl, Biphenyl, Naphthyl, 1-Naphthyl, 2-Naphthyl, Binaphthyl, Anthracenyl, Phenanthrenyl ,In-danyl, In-denyl, 1,2,3,4-Tetrahydronaphthyl, insbesondere Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Fluorphenyl, o-, m- oder p-Hydroxyphenyl, o-, m- oder p- Hydroxytolyl (kresolartige Reste), o-, m- oder p-Methoxyphenyl, o-, m- oder p-Aminophenyl, o-, m- oder p-Methylaminophenyl, o-, m- oder p-Dimethylaminophenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m- oder p-Acetylphenyl.

In einer bevorzugten Ausführungsform der Erfindung ist "Ar" ein ein-, zwei- oder dreikerniger aromatischer Carbozyklus mit 3 bis 20 C-Atomen, der unsubstituiert oder ein-, zwei- oder dreifach durch F, A, -(CY₂)ₚ-OY, -(CY₂)ₚ-NYY, -(CY₂)ₚ-COOY, -(CY₂)ₚ-CO-NYY oder -(CY₂)ₚ-NY-COY substituiert sein kann. Es ist besonders bevorzugt, dass "Ar" ein-, zwei- oder dreikerniges Aryl mit 5 bis 14 C-Atomen, das unsubstituiert oder ein-, zwei- oder dreifach durch F, Alk oder -(CY₂)ₚ-OY substituiert sein kann, bedeutet. Ganz besonders bevorzugte Beispiele sind Phenyl, Naphthyl, Anthracenyl oder Phenanthrenyl, das unsubstituiert oder ein- oder zweifach durch F, Alk oder -OAlk substituiert sein kann. Es versteht sich, dass die jeweiligen Bedeutungen von "Ar" unabhängig voneinander in den Radikalen einer erfindungsgemäßen Formel sind.

Das Radikal R1 bedeutet vorzugsweise A, Cyc, F, OY oder Ar, besonders bevorzugt A, Cyc, F, OH oder Ar.

Das Radikal R2 bedeutet vorzugsweise H, A, Cyc, F, OY oder Ar, besonders bevorzugt H, Alk, Cyc, F, OH oder Ar, ganz besonders bevorzugt H.

Die Indizes m, n und p bedeuten unabhängig voneinander vorzugsweise 0, 1 oder 2, besonders bevorzugt unabhängig voneinander 0 oder 1, ganz besonders bevorzugt unabhängig voneinander 0, höchst bevorzugt abhängig voneinander eine der oben genannten Bedeutungen.

Dementsprechend sind Gegenstand der Erfindung diejenigen Verbindungen der Formel (I) zur erfindungsgemäßen Verwendung, in denen mindestens eines der genannten Radikale eine der vorstehend angegebenen Bedeutungen hat. Nicht näher bezeichneten Radikale im Kontext einer Ausführungsform der Formel (I), Teilformel davon oder irgendeines Restes daran sollen die bei der Formel (I) angegebene Bedeutung haben, wie sie hierin offenbart ist, um die Aufgabe der Erfindung zu lösen. Das heißt, dass die genannten Radikale sämtliche ihnen zugeordneten Bedeutungen annehmen können, wie sie vor- oder nachstehend beschrieben sind, einschließlich jeglicher bevorzugter Ausführungsformen, ohne auf diese beschränkt zu sein und unabhängig von ihrem Auftreten in einem anderen bestimmten Kontext. Es versteht sich insbesondere, dass jede Ausführungsform eines bestimmten Radikals mit jeder Ausführungsform eines oder mehrerer anderer Radikale kombiniert werden kann.

In einer bevorzugten Ausgestaltung der vorliegenden Erfindung werden 2,2'-Furoin-Derivate der Formel (I) und/oder ihre physiologisch unbedenklichen Salze, Tautomere, Stereoisomere und/oder Solvate, einschließlich deren Mischungen in allen Verhältnissen, zur erfindungsgemäßen Verwendung bereitgestellt, worin
- R1: A, Cyc, F, OY oder Ar,
- R2: Y, Cyc, F, OH oder Ar,
- Y: H oder Alk,
- A: unverzweigtes oder verzweigtes Alkyl mit 1-18 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- oder -CΞC-Gruppe ersetzt sein können,
- Alk: unverzweigtes oder verzweigtes Alkyl mit 1-12 C-Atomen,
- Cyc: zyklisches Alkyl mit 3-8 C-Atomen, wobei unabhängig voneinander 1-3 H-Atome durch F und/oder unabhängig voneinander eine oder zwei benachbarte CH₂-Gruppen durch -O-, -N(Alk)- oder eine -CH=CH- Gruppe ersetzt sein können,
- Ar: ein-, zwei- oder dreikerniges Aryl mit 5 bis 14 C-Atomen, das unsubstituiert oder ein-, zwei- oder dreifach durch F, Alk oder -(CY₂)ₚ-OY substituiert sein kann, und
- m, n, p: unabhängig voneinander 0, 1 oder 2 bedeuten.

In einer besonders bevorzugten Ausgestaltung der vorliegenden Erfindung werden 2,2'-Furoin-Derivate der Formel (I) und/oder ihre physiologisch unbedenklichen Salze, Tautomere, Stereoisomere und/oder Solvate, einschließlich deren Mischungen in allen Verhältnissen, zur erfindungsgemäßen Verwendung bereitgestellt, worin
- R1: A, Cyc, F, OH oder Ar,
- R2: H,
- Y, A: unabhängig voneinander Alk,
- Alk: Methyl, Ethyl oder Trifluormethyl,
- Cyc: Cycloprenyl, Cyclobutenyl, Cyclopentenyl, Cyclopentadienyl, Cyclohexenyl, Cyclohexadienyl oder Cyclooctadienyl,
- Ar: Phenyl, Naphthyl, Anthracenyl oder Phenanthrenyl, das unsubstituiert oder ein- oder zweifach durch F, Alk oder -(CY₂)ₚ-OY substituiert sein kann, und
- m, n, p: unabhängig voneinander 0 oder 1 bedeuten.

In einer ganz besonders bevorzugten Ausgestaltung der vorliegenden Erfindung werden 2,2'-Furoin der Teilformel (IA) 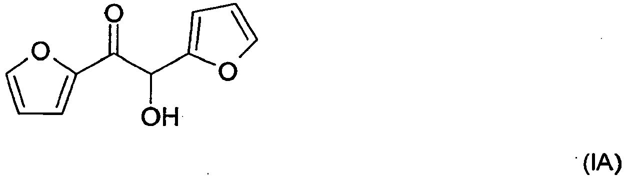 und/oder ihre physiologisch unbedenklichen Salze, Tautomere, Stereoisomere und/oder Solvate, einschließlich deren Mischungen in allen Verhältnissen, und/oder 1,2-Di-2-furanyl-2-hydroxy-2-phenyl-ethanon der Teilformel (IB) 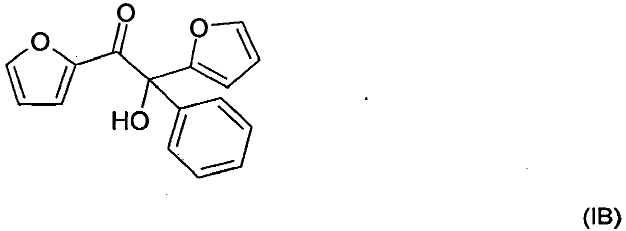 und/oder ihre physiologisch unbedenklichen Salze, Tautomere, Stereoisomere und/oder Solvate, einschließlich deren Mischungen in allen Verhältnissen,
erfindungsgemäß verwendet.

Höchst bevorzugt ist die erfindungsgemäße Verwendung von 2,2'-Furoin der Teilformel (IA), d.h. deren Verwendung zur nicht-therapeutischen Aufhellung von Haut, zur Hemmung von Tyrosinase in-vitro und/oder zur Verwendung in der Prophylaxe, Therapie und/oder Verlaufskontrolle von Pigmentstörungen der Haut.

Die Verbindungen der Formel (I) und auch die Ausgangsstoffe zu ihrer Herstellung werden nach an sich bekannten Methoden hergestellt, wie sie in der Literatur (z.B. in Standardwerken wie Houben-Weyl, Methoden der organischen Chemie, Georg-Thieme-Verlag, Stuttgart) beschrieben und/oder Fachmann bekannt sind, sowie unter Reaktionsbedingungen, die für die genannten Umsetzungen bekannt und geeignet sind. Dabei kann man auch von an sich bekannten, hier nicht näher erwähnten Varianten Gebrauch machen.

Die Reaktionszeit liegt je nach angewendeten Bedingungen zwischen einigen Minuten und 14 Tagen, die Reaktionstemperatur zwischen -78°C und 150°C, normalerweise zwischen -20°C und 100°C, besonders bevorzugt zwischen 0°C und 70°C.

Die Umsetzung erfolgt in einem inerten Lösungsmittel und in der Regel in Gegenwart eines säurebindenden Mittels, vorzugsweise einer organischen Base wie DIPEA, Triethylamin, Dimethylanilin, Pyridin, Chinolin, Piperidin oder Diethanolamin. Auch der Zusatz eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein. Als Basen eignen sich Metalloxide, wie z.B. Aluminiumoxid, Alkalimetallhydroxide (darunter Kaliumhydroxid, Natriumhydroxid und Lithiumhydroxid), Erdalkalimetallhydroxide (z.B. Bariumhydroxid und Calciumhydroxid) und Alkalimetallalkoholate (z.B. Kaliumethanolat und Natriumpropanolat).

Als inerte Lösungsmittel eignen sich u.a. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Besonders bevorzugt sind THF, Glykolether, wie Ethylenglycolmonomethylether, Dichlormethan und/oder DMF.

Das Verfahren und die anschließende Aufarbeitung des Reaktionsgemisches können grundsätzlich als Batch-Reaktion oder in kontinuierlicher Reaktionsweise durchgeführt werden. Die kontinuierliche Reaktionsweise umfasst z.B. die Reaktion in einem kontinuierlichen Rührkesselreaktor, einer Rührkesselkaskade, einem Schlaufen- oder Querstromreaktor, einem Strömungsrohr oder in einem Mikroreaktor. Die Aufarbeitung der Reaktionsgemische erfolgt wahlweise, je nach Bedarf, durch Filtration über feste Phasen, Chromatographie, Separation zwischen unmischbaren Phasen (z. B. Extraktion), Adsorption an festen Trägern, Abdestillieren von Lösungsmitteln und/oder azeotropen Gemischen, selektive Destillation, Sublimation, Kristallisation, Co-Kristallisation oder durch Nanofiltration an Membranen.

Die Verbindungen der Formel (I) können vorzugsweise erhalten werden, indem man eine Benzoin-Kondensation durchführt. Gegenstand der vorliegenden Erfindung ist damit auch ein Verfahren zum Herstellen und/oder Herrichten von Verbindungen der Formel (I), Teilformeln davon und/oder ihrer physiologisch unbedenklichen Salze, Tautomere, Stereoisomere und/oder Solvate, einschließlich deren Mischungen in allen Verhältnissen, mit den folgenden Schritten:
(a) Umsetzen eines Furfural-Derivates der Formel (II) 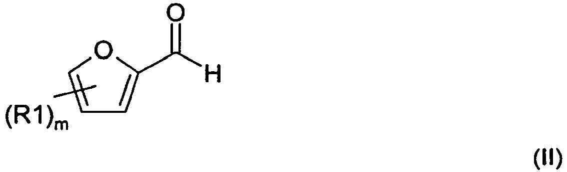 worin R1 und m die oben angegebene Bedeutung aufweisen,
   mit einem Furfural-Derivat der Formel (III) 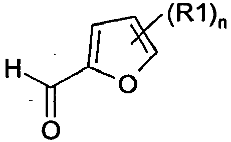 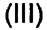 worin R1 und n die oben angegebene Bedeutung aufweisen,
   in einer Benzoin-Addition unter Erhalt von Verbindungen der Formel (I) 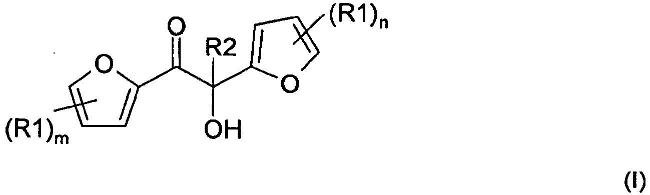 worin R1, m und n die oben angegebene Bedeutung aufweisen,
   und optional
(b) Umwandeln einer Base oder Säure der Verbindungen der Formel (I) in eines ihrer physiologisch unbedenklichen Salze,
   und/oder
(c) augenfälliges Herrichten der Verbindungen der Formel (I) oder eines ihrer physiologisch unbedenklichen Salze zur erfindungsgemäßen Verwendung.

Es versteht sich vorzugsweise, dass lediglich der Schritt (b) optional ist, während der Schritt (c) obligatorisch ist und entweder auf Schritt (a) oder den optionalen Schritt (b) folgt. In einer anderen Ausgestaltung betrifft die Erfindung auch ein Verfahren zum Herstellen und ggf. Herrichten von Verbindungen der Formel (I), Teilformeln davon und/oder ihrer physiologisch unbedenklichen Salze, Tautomere, Stereoisomere und/oder Solvate, einschließlich deren Mischungen in allen Verhältnissen, zur Verwendung in der Prophylaxe, Therapie und/oder Verlaufskontrolle von Pigmentstörungen der Haut, mit den oben genannten Schritten, wobei Schritt (c) optional ist, aber vorzugsweise obligatorisch.

Die Ausgangsverbindungen sind in der Regel bekannt. Sind sie neu, so können sie nach an sich bekannten Methoden hergestellt werden. Die Verbindungen der Formeln (II) und (III) können nach bekannten Methoden bereitgestellt werden. Fall gewünscht, können die Ausgangsstoffe in-situ gebildet werden, so dass man sie aus dem Reaktionsgemisch nicht isoliert, sondern sofort weiter zu den erfindungsgemäßen Verbindungen umsetzt. Es ist ebenso möglich, die Reaktion schrittweise durchzuführen.

Die genannten erfindungsgemäßen Verbindungen lassen sich in ihrer endgültigen Nichtsalzform verwenden. Andererseits umfasst die vorliegende Erfindung auch die Verwendung dieser Verbindungen in Form ihrer physiologisch unbedenklichen Salze, die von verschiedenen organischen und anorganischen Säuren und Basen nach fachbekannten Vorgehensweisen abgeleitet werden können. Physiologisch unbedenkliche Salzformen der Verbindungen der Formel (I) sowie ihrer Teilformeln werden größtenteils konventionell hergestellt. Sofern die Verbindungen eine Carbonsäuregruppe enthalten, lässt sich eines ihrer geeigneten Salze dadurch bilden, dass man die Verbindung mit einer geeigneten Base zum entsprechenden Basenadditionssalz umsetzt. Solche Basen sind zum Beispiel Alkalimetallhydroxide (z.B. Kaliumhydroxid, Natriumhydroxid und Lithiumhydroxid), Erdalkalimetallhydroxide (z.B. Bariumhydroxid und Calciumhydroxid), Alkalimetallalkoholate (z.B. Kaliumethanolat und Natriumpropanolat) sowie verschiedene organische Basen wie Piperidin, Diethanolamin und N-Methylglutamin. Eine Base der Formel (I) sowie deren Teilformeln kann mit einer Säure in das zugehörige Säureadditionssalz übergeführt werden, beispielsweise durch Umsetzung äquivalenter Mengen der Base und der Säure in einem inerten Lösungsmittel, wie z.B. Ethanol, und Eindampfen im Anschluss. Für diese Umsetzung kommen insbesondere Säuren in Frage, die physiologisch unbedenkliche Salze liefern, wie z.B. Halogenwasserstoffe (z.B. Chlorwasserstoff, Bromwasserstoff oder Jodwasserstoff), andere Mineralsäuren und ihre entsprechenden Salze (z.B. Sulfat, Nitrat oder Phosphat und dergleichen), Alkyl- und Monoarylsulfonaten (z.B. Ethansulfonat, Toluolsulfonat und Benzolsulfonat) sowie andere organische Säuren und ihre entsprechenden Salze (z.B. Acetat, Trifluoracetat, Tartrat, Maleat, Succinat, Citrat, Benzoat, Salicylat, Ascorbat und dergleichen. Salze mit physiologisch bedenklichen Säuren, z.B. Pikrate, können zur Isolierung und/oder Aufreinigung der Verbindungen der Formel (I) verwendet werden.

Es versteht sich, dass die Salze im Sinne der Erfindung auf den Carboxylatgruppen, wie sie in den Verbindungen der Formel (I) enthalten sind, basieren können, indem deren azides Proton gegen ein Metall ausgetauscht werden kann. Alternativ können die Salze im Sinne der Erfindung auch auf Verbindungen der Formel (I) basieren, bei denen an der Alkoholfunktion der Furoin-Derivate das Proton gegen ein Ion getauscht ist. Das können einwertige Ionen, wie z.B. Li⁺, Na⁺, K⁺ oder NH₄⁺ sein, aber auch mehrwertige Ionen, wodurch Salze vom Typ (Furoin)₂M oder (Furoin)₃M₂ etc. entstehen.

Im Hinblick auf das oben Gesagte sieht man, dass unter dem Ausdruck "physiologisch unbedenkliches Salz" im vorliegenden Zusammenhang ein Wirkstoff zu verstehen ist, der eine Verbindung der Formel (I) in der Form eines ihrer Salze enthält, insbesondere dann, wenn diese Salzform dem Wirkstoff im Vergleich zu der freien Form des Wirkstoffs verbesserte kosmetische und/oder pharmakokinetische Eigenschaften verleiht. Die physiologisch unbedenkliche Salzform des Wirkstoffs kann diesem Wirkstoff auch erst eine gewünschte kosmetische und/oder pharmakokinetische Eigenschaft verleihen und kann sogar die Pharmakodynamik dieses Wirkstoffs in Bezug auf seine therapeutische Wirksamkeit im Körper positiv beeinflussen. Bevorzugte Salze im Sinne der Erfindung sind die Lithium-, Natrium-, Kalium-, Magnesium-, Calcium-, Zink-, Kupfer- und AmmoniumSalze der Verbindungen der Formel (I).

Die Verbindungen der Formel (I) können aufgrund ihrer Molekülstruktur chiral sein und dementsprechend in verschiedenen enantiomeren Formen auftreten. Sie können daher in racemischer oder in optisch aktiver Form vorliegen. Da sich die kosmetische und/oder pharmazeutische Wirksamkeit der Racemate bzw. der Stereoisomeren der Verbindungen der Formel (I) unterscheiden kann, mag es wünschenswert sein, die Enantiomere zu verwenden. In diesen Fällen kann das Endprodukt oder bereits das Zwischenprodukt in enantiomere Verbindungen, durch dem Fachmann bekannte chemische oder physikalische Maßnahmen, aufgetrennt oder bereits als solche bei der Synthese eingesetzt werden.

Es versteht sich, dass das augenfällige Herrichten auf die Verwendung im Sinne der Erfindung gerichtet ist, d.h. zur Verwendung bei der nicht-therapeutischen Aufhellung von Haut, zur Verwendung bei der Hemmung von Tyrosinase in-vitro, zur Verwendung in der Prophylaxe, Therapie und/oder Verlaufskontrolle von Pigmentstörungen der Haut und/oder zur Verminderung von Kontrastunterschieden im Haufarbton, die durch unterschiedlich stark pigmentierte Areale hervorgerufen werden. Die augenfällige oder sinnfällige Herrichtung kann beispielsweise bestehen in: a) besonderer Gestaltung des Stoffs oder der Sache, also wenn diese so individualisiert sind, dass eine Eignung für den patentgemäßen Gebrauch klar ersichtlich ist; b) Beigabe einer Gebrauchsanleitung (z.B. Beipackzettel) beim Vertrieb; c) Formulierung, Konfektionierung, Dosierung und gebrauchsfertige Verpackung; d) Therapieplan, Dosisempfehlung; e) Benutzung einer verwendungsspezifischen Warenbezeichnung (Schulte/Kühnen, PatG, 8. Auflage, § 14 Rdn 101).

Ein weiterer Gegenstand der vorliegenden Erfindung ist eine Zubereitung enthaltend mindestens eine Verbindung der Formel (I), Teilformel (IA), (IB), (IC) und/oder ihrer physiologisch unbedenklichen Salze, Tautomere, Stereoisomere und/oder Solvate, einschließlich deren Mischungen in allen Verhältnissen, zur erfindungsgemäßen Verwendung und einen für kosmetische, pharmazeutische oder dermatologische Anwendungen geeigneten Träger und optional physiologisch unbedenkliche Hilfsstoffe und/oder Füllstoffe. Im Sinne der vorliegenden Erfindung wird neben dem Begriff "Zubereitung" gleichbedeutend auch der Begriff "Mittel", "Zusammensetzung" oder "Formulierung" verwendet. Bei den Zubereitungen handelt es sich dabei üblicherweise um topisch anwendbare Zubereitungen, wie z.B. kosmetische oder dermatologische Formulierungen oder Medizinprodukte. Topisch anwendbar bedeutet im Sinne der Erfindung, dass die Zubereitung äußerlich und örtlich angewendet wird, d.h. dass die Zubereitung beispielsweise geeignet sein muss, um auf die Haut aufgetragen werden zu können. Die Zubereitungen enthalten in diesem Fall einen kosmetisch, pharmazeutisch oder dermatologisch geeigneten Träger und je nach gewünschtem Eigenschaftsprofil optional weitere geeignete Inhaltsstoffe. Bevorzugt werden die topischen Zubereitungen als kosmetische oder dermatologische Zubereitung eingesetzt, insbesondere bevorzugt als kosmetische Zubereitung. Geeignete Trägerstoffe sowie Hilfs- oder Füllstoffe sind im nachfolgenden Teil ausführlich beschrieben.

Die Zubereitungen können die vor- und/oder nachstehend genannten notwendigen oder optionalen Bestandteile umfassen oder enthalten, daraus im Wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden. Weitere bevorzugte Kombinationen von Ausführungsformen sind in den Ansprüchen offenbart.

In der Zubereitung der Erfindung sind die Verbindung der Formel (I), wie zuvor angegeben und auch als bevorzugt beschrieben, und/oder ihre physiologisch unbedenklichen Salze, Tautomere, Stereoisomere und/oder Solvate, einschließlich deren Mischungen in allen Verhältnissen, in einer Menge von 0,0001 bis 20 Gew.-%, bezogen auf das Gesamtgewicht der Zubereitung, enthalten. Bevorzugt wird eine Menge von 0,0001 bis 15 Gew.-% eingesetzt, besonders bevorzugt von 0,0001 bis 10 Gew.-%, ganz besonders bevorzugt von 0,0001 bis 5 Gew.-%. Dabei bereitet es dem Fachmann keinerlei Schwierigkeiten die Mengen abhängig von der beabsichtigten Wirkung der Zubereitung entsprechend auszuwählen.

Weiterhin empfiehlt es sich, dass in der Zubereitung der Erfindung die Verbindung der Formel (I), deren Teilformeln und/oder ihre physiologisch unbedenklichen Salze, Tautomere, Stereoisomere und/oder Solvate, einschließlich deren Mischungen in allen Verhältnissen, in Kombination mit mindestens einem weiteren Wirkstoff enthalten sind. Der weitere Wirkstoff ist vorzugsweise ausgewählt aus der Gruppe von UV-Filtern, Antioxidantien, Vitaminen, hautaufhellenden Wirkstoffen, Anti-Aging-Wirkstoffen, anti-inflammatorischen Wirkstoffen, antimikrobiellen Wirkstoffen, Wirkstoffen zur Verbesserung des Feuchtegehaltes der Haut (Hautfechteregulatoren), Anti-Cellulite-Wirkstoffen, Anti-Falten-Wirkstoffen, Anti-Schuppen-Wirkstoffen, Anti-Akne-Wirkstoffen, Deodorants, Pigmenten und Selbstbräunungssubstanzen, besonders bevorzugt aus der Gruppe von UV-Filtern, Antioxidantien, Vitaminen, hautaufhellenden Wirkstoffen, Anti-Aging-Wirkstoffen und Anti-Cellulite-Wirkstoffen.

Erfindungsgemäß bevorzugte Zubereitungen enthalten neben mindestens einer Verbindung der Formel (I) auch einen oder mehrere UV-Filter. Prinzipiell kommen alle UV-Filter für eine Kombination mit den Verbindungen der Formel (I) oder Teilformeln davon in der erfindungsgemäßen Zubereitung in Frage. Besonders bevorzugt sind solche UV-Filter, deren physiologische Unbedenklichkeit bereits nachgewiesen ist. Sowohl für UVA- wie auch UVB-Filter gibt es viele aus der Fachliteratur bekannte und bewährte Substanzen. Die in den nachfolgenden Listen aufgeführten Verbindungen sind nur als Beispiele aufzufassen. Selbstverständlich können auch andere UV-Filter verwendet werden.

Bevorzugte Zubereitungen können neben den Verbindungen nach Formel (I) sowie den gegebenenfalls anderen Inhaltsstoffen organische UV-Filter enthalten, sogenannte hydrophile oder lipophile Sonnenschutzfilter, die im UVA-Bereich und/oder UVB-Bereich und/oder IR- und/oder VIS-Bereich (Absorber) wirksam sind. Diese Substanzen können insbesondere unter p-Aminobenzoesäurederivaten, Salicylsäurederivaten, β,β-Diphenylacrylatderivaten, Campherderivaten, Triazinderivaten, Zimtsäurederivaten sowie polymeren Filtern und Siliconfiltern, die in der Anmeldung WO 93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt. Insbesondere für eine Kombination geeignet sind:
- para-Aminobenzoesäure und deren Derivate: PABA, Ethyl PABA, Ethyl dihydroxypropyl PABA, Ethylhexyl dimethyl PABA, z.B. vetrieben unter dem Namen "Escalol 507" von der Fa. ISP, Glyceryl PABA, PEG-25 PABA, z.B. vertrieben unter dem Namen "Uvinul P25" von der Fa. BASF.
- Salicylate: Homosalate, vertrieben unter dem Namen "Eusolex HMS" von der Fa. Merck; Ethylhexyl salicylate, z.B. vertrieben unter dem Namen "Neo Heliopan OS" von der Fa. Symrise; Dipropylene glycol salicylate, z.B. vertrieben unter dem Namen "Dipsal" von der Fa. Scher; TEA salicylate, z.B. vertrieben unter dem Namen "Neo Heliopan TS" von der Fa. Symrise.
- β,β-Diphenylacrylate Derivate: Octocrylene, z.B. vertrieben unter dem Namen "Eusolex® OCR" von der Firma Merck; "Uvinul N539" von der Fa. BASF; Etocrylene, z.B. vertrieben unter dem Namen "Uvinul N35" von der Fa. BASF.
- Benzophenon Derivate: Benzophenon-1, z.B. vertrieben unter dem Namen "Uvinul 400"; Benzophenon-2, z.B. vertrieben unter dem Namen "Uvinul D50"; Benzophenon-3 oder Oxybenzon, z.B. vertrieben unter dem Namen "Uvinul M40"; Benzophenon-4, z.B. vertrieben unter dem Namen "Uvinul MS40"; Benzophenon-9, z.B. vertrieben unter dem Namen "Uvinul DS-49" von der Fa. BASF; Benzophenon-5, Benzophenon-6, z.B. vertrieben unter dem Namen "Helisorb 11" von der Fa. Norquay; Benzophenon-8, z.B. vertrieben unter dem Namen "Spectra-Sorb UV-24" von der Fa. American Cyanamid; Benzophenon-12 n-hexyl 2-(4-diethylamino-2-hydroxybenzoyl) benzoate oder 2-Hydroxy-4-methoxybenzophenon, vertrieben von der Fa. Merck, Darmstadt, unter dem Namen Eusolex® 4360.
- Benzylidencampher Derivate: 3-Benzylidencamphor, z.B. vertrieben unter dem Namen "Mexoryl SD" von der Fa. Chimex; 4-Methylbenzylidencamphor, z.B. vertrieben unter dem Namen "Eusolex 6300" von der Fa. Merck; Benzylidencamphorsulfonsäure, z.B. vertrieben unter dem Namen "Mexoryl SL" von der Fa. Chimex; Camphor benzalkonium methosulfate, z.B. vertrieben unter dem Namen "Mexoryl SO" von der Fa. Chimex; Terephthalylidendicamphorsulfonsäure, z.B. vertrieben unter dem Namen "Mexoryl SX" von der Fa Chimex; Polyacrylamidomethylbenzylidencamphor, vertrieben unter dem Namen "Mexoryl SW" von der Fa. Chimex.
- Phenylbenzimidazol Derivate: Phenylbenzimidazolsulfonsäure, z.B. vertrieben unter dem Namen "Eusolex 232" von der Fa. Merck; Dinatrium phenyl dibenzimidazole tetrasulfonat, z.B. vertrieben unter dem Namen "Neo Heliopan AP" von der Fa. Symrise.
- Phenylbenzotriazol Derivate: Drometrizol trisiloxane, z.B. vertrieben unter dem Namen "Silatrizole" von der Fa. Rhodia Chimie; Methylenebis(benzotriazolyl)tetramethylbutylphenol in fester Form, z.B. vertrieben unter dem Namen "MIXXIM BB/100" von der Fa. Fairmount Chemical, oder in mikronisierter Form als wässrige Dispersion, z.B. vertrieben unter dem Namen 'Tinosorb M" von der Fa. BASF.
- Triazin Derivate: Ethylhexyltriazone, z.B. vertrieben unter dem Namen "Uvinul T150" von der Fa. BASF; Diethylhexylbutamidotriazone, z.B. vertrieben unter dem Namen "Uvasorb HEB" von der Fa. Sigma 3V; 2,4,6-tris(diisobutyl 4'-aminobenzalmalonate)-s-triazine oder 2,4,6-Tris-(biphenyl)-1,3,5-triazine, vertrieben als Tinosorb A2B von der Fa. BASF; 2,2'-[6-(4-methoxyphenyl)-1,3,5-triazine-2,4-diyl]bis[5-(2-ethylhexyl)oxy]-phenol, vertrieben als Tinosorb S von der Fa. BASF; N2,N4-bis[4-[5-(1,1-dimethylpropyl)-2-benzoxazolyl]phenyl]-N6-(2-ethylhexyl)-1,3,5-triazin-2,4,6-triamin, vertrieben als Uvasorb K 2A von der Fa. Sigma 3V.
- Anthranilin Derivate: Menthyl anthranilate, z.B. vertrieben unter dem Namen "Neo Heliopan MA" von der Fa. Symrise.
- Imidazol Derivate: Ethylhexyldimethoxybenzylidenedioxoimidazoline propionat.
- Benzalmalonat Derivate: Polyorganosiloxane enthaltend funktionelle Benzalmalonat-Gruppen, wie z.B. Polysilicone-15, z.B. vertrieben unter dem Namen "Parsol SLX" von der Hoffmann LaRoche.
- 4,4-Diarylbutadien Derivate: 1,1-Dicarboxy(2,2'-dimethylpropyl)-4,4-diphenylbutadiene.
- Benzoxazol Derivate: 2,4-bis[5-(1-dimethylpropyl)benzoxazol-2-yl(4-phenyl) imino]-6-(2-ethylhexyl)imino-1,3,5-triazine, z.B. vertrieben unter dem Namen Uvasorb K2A von der Fa. Sigma 3V und Mischungen dieses enthaltend.
- Piperazinderivate: beispielsweise die Verbindung 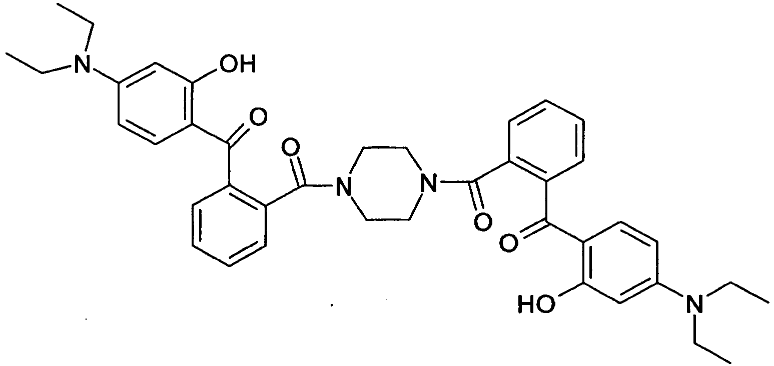 oder die UV-Filter der folgenden Strukturen

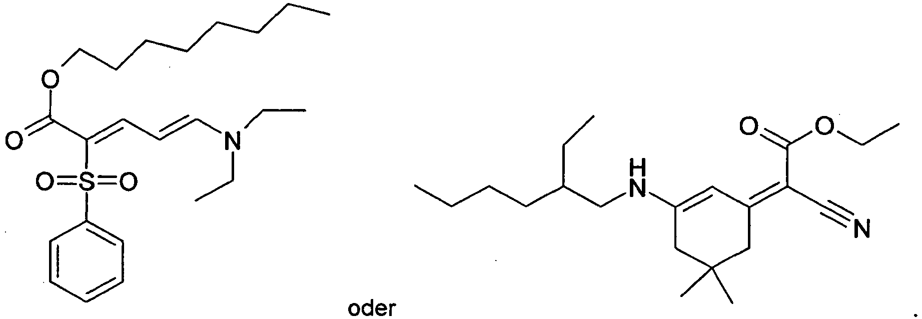

Es können auch UV-Filter auf Basis von Polysiloxancopolymeren mit einer statistischen Verteilung gemäß nachfolgender Formel verwendet werden, wobei z.B. a = 1,2; b= 58 und c=2,8 sind:

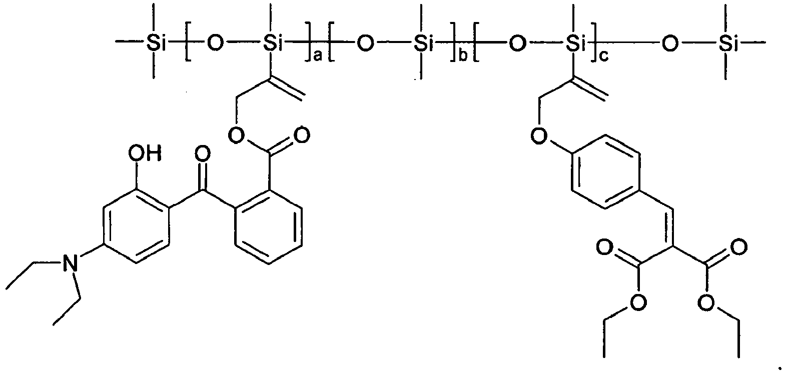

Geeignete organische UV-schützende Substanzen sind bevorzugt aus der folgenden Liste auszuwählen: Ethylhexyl salicylate, Phenylbenzimidazolsulfonsäure, Benzophenon-3, Benzophenon-4, Benzophenon-5, n-Hexyl 2-(4-diethylamino-2-hydroxybenzoyl)benzoate, 4-Methylbenzylidencamphor, Terephthalylidendicamphorsulfonsäure, Dinatrium phenyldibenzimidazoltetrasulfonat, Methylenbis(benzotriazolyl)tetramethylbutylphenol, Ethylhexyltriazone, Diethylhexylbutamidotriazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)-benzoxazol-2-yl(4-phenyl) imino]-6-(2-ethylhexyl)imino-1,3,5-triazine und Mischungen davon.

Diese organischen UV-Filter werden in der Regel in einer Menge von 0,01 bis 20 Gewichtsprozent, vorzugsweise 1 bis 20 Gew.-%, in Formulierungen eingearbeitet.

Die Zubereitungen können neben den Verbindungen der Formel (I) sowie den gegebenenfalls anderen organischen UV-Filtern, wie zuvor beschrieben, weitere anorganische UV-Filter, sogenannte partikuläre UV-Filter enthalten. Diese Kombinationen mit partikulären UV-Filtern sind sowohl als Pulver als auch als Dispersion oder Paste der folgenden Typen möglich. Hierbei sind sowohl solche aus der Gruppe der Titandioxide, wie z.B. beschichtetes Titandioxid (z.B. Eusolex^{®} T-2000, Eusolex^{®} T-AQUA, Eusolex^{®} T-AVO, Eusolex^{®} T-OLEO), Zinkoxide (z.B. Sachtotec^{®}), Eisenoxide oder auch Ceroxide und/oder Zirkonoxide bevorzugt. Ferner sind auch Kombinationen mit pigmentärem Titandixoxid oder Zinkoxid möglich, wobei die Partikelgröße dieser Pigmente größer oder gleich 200 nm ist, beispielsweise Hombitan^{®} FG oder Hombitan^{®} FF-Pharma.

Weiter kann es bevorzugt sein, wenn die Zubereitungen anorganische UV-Filter enthalten, die mit üblichen Methoden, wie beispielsweise in Cosmetics & Toiletries 1990, 105, 53 beschrieben, nachbehandelt wurden. Hierbei können eine oder mehrere der folgenden Nachbehandlungskomponenten gewählt sein: Aminosäuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Bevorzugt eingesetzte partikuläre UV-Filter sind dabei:
- Unbehandelte Titandioxide, wie z.B. die Produkte Microtitanium Dioxide MT 500 B der Fa. Tayca; Titandioxd P25 der Fa. Degussa;
- Nachbehandelte mikronisierte Titandioxide mit Aluminiumoxid- und Siliciumdioxid-Nachbehandlung, wie z.B. das Produkt "Microtitanium Dioxide MT 100 SA der Tayca, oder das Produkt "Tioveil Fin" der Fa. Uniqema;
- Nachbehandelte mikronisierte Titandioxide mit Aluminiumoxid- und/oder Aluminiumstearate/laurate-Nachbehandlung, wie z.B. Microtitanium Dioxide MT 100 T der Fa. Tayca; Eusolex T-2000 der Firma Merck;
- Nachbehandelte mikronisierte Titandioxide mit Eisenoxid- und/oder Eisenstearate-Nachbehandlung, wie z.B. das Produkt "Microtitanium Dioxide MT 100 F" der Fa. Tayca;
- Nachbehandelte mikronisierte Titandioxide mit Siliciumdioxid-, Aluminiumoxid- und Silicon-Nachbehandlung, wie z.B. das Produkt "Microtitanium Dioxide MT 100 SAS",der Fa. Tayca;
- Nachbehandelte mikronisierte Titandioxide mit Natrumhexametaphosphat, wie z.B. das Produkt "Microtitanium Dioxide MT 150 W" der Fa. Tayca.

Die zur Kombination eingesetzten behandelten mikronisierten Titandioxide können auch nachbehandelt sein mit:
- Octyltrimethoxysilane, wie z.B. das Produkt Tego Sun T 805 der Fa. Degussa;
- Siliciumdioxid, wie z.B. das Produkt Parsol T-X der Fa. DSM;
- Aluminiumoxid und Stearinsäure, wie z.B. das Produkt UV-Titan M160 der Fa. Sachtleben;
- Aluminium und Glycerin, wie z.B. das Produkt UV-Titan der Fa. Sachtleben;
- Aluminium und Silikonölen, wie z.B. das Produkt UV-Titan M262 der Fa. Sachtleben;
- Natriumhexamethaphosphat und Polyvinylpyrrolidon;
- Polydimethylsiloxane, wie z.B. das Produkt 70250 Cardre UF TiO2SI3" der Fa. Cardre;
- Polydimethylhydrogensiloxane, wie z.B. das Produkt Microtitanium Dioxide USP Grade Hydrophobic" der Fa. Color Techniques.

Ferner kann auch die Kombination mit folgenden Produkten vorteilhaft sein:
- Unbehandelte Zinkoxide, wie z.B. das Produkt Z-Cote der Fa. BASF (Sunsmart), Nanox der Fa. Elementis;
- Nachbehandelte Zinkoxide, wie z.B. die folgenden Produkte:
   o "Zinc Oxide CS-5" der Fa. Toshibi (ZnO nachbehandelt mit Polymethylhydrogenosiloxan);
   ∘ Nanogard Zinc Oxide FN der Fa. Nanophase Technologies;
   o "SPD-Z1" der Fa Shin-Etsu (ZnO nachbehandelt mit einem Silikongepfropften Acrylpolymer, dispergiert in Cyclodimethylsiloxane);
   o "Escalol Z100" der Fa ISP (Aluminiumoxid-nachbehandeltes ZnO, dispergiert in einer Ethylhexyl methoxycinnamate/PVP-hexadecene/methicone copolymer Mischung);
   o "Fuji ZNO-SMS-10" der Fa. Fuji Pigment (ZnO nachbehandelt mit Siliciumdioxid und Polymethylsilesquioxan);
   ∘ Unbehandeltes Ceroxid-Mikropigment, z.B. mit der Bezeichnung "Colloidal Cerium Oxide" der Fa Rhone Poulenc;
   o Unbehandelte und/oder nachbehandelte Eisenoxide mit der Bezeichnung Nanogar der Fa. Arnaud.

Beispielhaft können auch Mischungen verschiedener Metalloxide, wie z.B. Titandioxid und Ceroxid mit und ohne Nachbehandlung eingesetzt werden, wie z.B. das Produkt Sunveil A der Fa. Ikeda. Außerdem können auch Mischungen von Aluminiumoxid-, Siliciumdioxid- und Silikonnachbehandelten Titandioxid / Zinkoxid-Mischungen, wie z.B. das Produkt UV-Titan M261 der Fa. Sachtleben eingesetzt werden.

Diese anorganischen UV-Filter werden in der Regel in einer Menge von 0,1 bis 25 Gewichtsprozent, vorzugsweise 2 bis 10 Gew.-%, in die Zubereitungen eingearbeitet.

Durch Kombination von einer oder mehrerer der genannten Verbindungen mit UV-Filterwirkung kann die Schutzwirkung gegen schädliche Einwirkungen der UV-Strahlung optimiert werden.

Alle genannten UV-Filter können auch in verkapselter Form eingesetzt werden. Insbesondere ist es von Vorteil organische UV-Filter in verkapselter Form einzusetzen. Dabei sind die Kapseln in erfindungsgemäß einzusetzenden Zubereitungen vorzugsweise in solchen Mengen enthalten, die gewährleisten, dass die verkapselten UV-Filter in den oben angegebenen Gewichtsprozentverhältnissen in der Zubereitung vorliegen.

Eine weitere bevorzugte Ausführungsform der Erfindung betrifft die Kombination der Verbindungen der Formel (I) mit mindestens einer Substanz, die der Aufrechterhaltung und/oder der Verbesserung des Feuchtigkeitsgehaltes der Haut dient. Diese Substanzen können, ohne dass dies als Einschränkung aufgefasst werden soll, unter anderem auch Substanzen sein, die zu den sogenannten natürlichen Feuchtigkeitsfaktoren (natural moisturizing factors) gehören, wie z.B. 2-Oxopyrrolidine 5-carbonsäure.

In einer weiteren bevorzugten Ausgestaltung der Erfindung enthält die Zubereitung ein oder mehrere Antioxidantien und/oder ein oder mehrere Vitamine. Durch den Einsatz von Antioxidantien kann eine schützende Wirkung gegen oxidativen Stress bzw. gegen die Einwirkung von Radikalen generell erzielt werden, wobei es dem Fachmann keinerlei Schwierigkeiten bereitet geeignet schnell oder zeitverzögert wirkende Antioxidantien auszuwählen. Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide, wie z.B. D,L-Carnosin, D-Carnosin, L-Carnosin und deren Derivate (z.B. Anserin), Carotinoide, Carotine (wie z.B. α-Carotin, β-Carotin, Lycopin) und deren Derivate, Chlorogensäure und deren Derivate, Liponsäure und deren Derivate (wie z.B. Dihydroliponsäure), Aurothioglucose, Propylthiouracil und andere Thiole (wie z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (wie z.B. Ester, Ether, Peptide, Lipide, Nukleotide, Nukleoside und Salze) sowie Sulfoximinverbindungen (wie z.B. Buthioninsulfoximine, Homocysteinsulfoximin, Buthioninsulfone, Penta-, Hexa-, Heptathioninsulfoximin) in sehr geringen verträglichen Dosierungen (wie z.B. pmol bis µmol/kg), ferner (Metall-) Chelatoren, (wie z.B. α-Hydroxyfettsäuren, Palmitinsäure, Phytinsäure, Lactoferrin), α-Hydroxysäuren (wie z.B. Zitronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (wie z.B. Ascorbylpalmitat, Magnesium-Ascorbylphosphat, Ascorbylacetat), Tocopherole und Derivate (wie z.B. Vitamin-E-Acetat), Vitamin A und Derivate (wie z.B. Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (wie z.B. ZnO, ZnSO₄), Selen und dessen Derivate (wie z.B. Selenmethionin), Stilbene und deren Derivate (wie z.B. Stilbenoxid, trans-Stilbenoxid). Weitere geeignete Antioxidantien sind auch in WO 2006/111233 und WO 2006/111234 beschrieben.

Geeignete Antioxidantien sind auch Verbindungen der allgemeinen Formeln A oder B 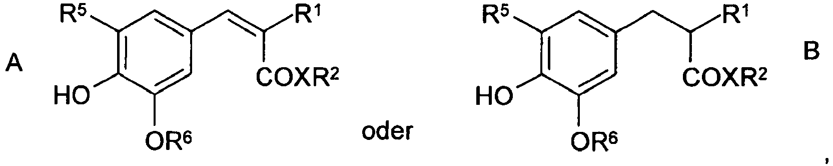 worin
- R¹: -C(O)CH₃, -CO₂R³, -C(O)NH₂ oder -C(O)N(R⁴)₂,
- X: O oder NH,
- R²: lineares oder verzweigtes Alkyl mit 1 bis 30 C-Atomen,
- R³: lineares oder verzweigtes Alkyl mit 1 bis 20 C-Atomen,
- R⁴: H oder lineares oder verzweigtes Alkyl mit 1 bis 8 C-Atomen,
- R⁵: H, lineares oder verzweigtes Alkyl mit 1 bis 8 C-Atomen oder lineares oder verzweigtes Alkoxy mit 1 bis 8 C-Atomen, und
- R⁶: lineares oder verzweigtes Alkyl mit 1 bis 8 C-Atomen bedeutet.

Bevorzugt sind Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (wie z.B. Oxynex^{®} ST Liquid) und/oder 2-(4-Hydroxy-3,5-dimethoxybenzy)-malonsäure-bis-(2-ethylhexyl)ester (wie z.B. RonaCare^{®} AP).

Mischungen von Antioxidantien sind ebenfalls zur Verwendung in den erfindungsgemäßen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (wie z.B. Oxynex^{®} AP), natürliche Tocopherole, L-(+)-Ascorbylpalmitat, L-(+)-Ascorbinsäure und Zitronensäure (wie z.B. Oxynex^{®} K LIQUID), Tocopherolextrakte aus natürlichen Quellen, L-(+)-Ascorbylpalmitat, L-(+)-Ascorbinsäure und Zitronensäure (wie z.B. Oxynex^{®} L LIQUID), DL-α-Tocopherol, L-(+)-Ascorbylpalmitat, Zitronensäure und Lecithin (wie z.B. Oxynex^{®} LM) oder Butylhydroxytoluol (BHT), L-(+)-Ascorbylpalmitat und Zitronensäure (wie z.B. Oxynex^{®} 2004). Derartige Antioxidantien werden mit Verbindungen der Formel (I) oder Teilformeln davon in solchen Zusammensetzungen üblicherweise in Gewichtsprozentverhältnissen im Bereich von 1000:1 bis 1:1000, bevorzugt in Gewichtsprozentverhältnissen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich Lemanska et al., Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. Rice-Evans et al., Trends in Plant Science 1997, 2(4), 152-159). Lemanska et al., Free Radical Biology & Medicine 2001, 31(7), 869-881, untersuchen die pH-Abhängigkeit der antioxidanten Wirkung von Hydroxyflavonen. Über den gesamten pH-Bereich zeigt Quercetin die höchste Aktivität der untersuchten Strukturen.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K1, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin. Vitamine werden mit den Zubereitungen üblicherweise bei kosmetischer Anwendung in Bereichen von 0,01 bis 5 Gew.-%, bezogen auf das Gesamtgewicht, zugesetzt. Ernährungsphysiologische Anwendungen orientieren sich am jeweiligen empfohlenen Vitaminbedarf.

Die erfindungsgemäßen Zubereitungen können auch ein oder mehrere weitere hautaufhellende Wirkstoffe oder synonym Depigmentierungswirkstoffe enthalten. Hautaufhellende Wirkstoffe können prinzipiell alle dem Fachmann bekannte Wirkstoffe sein. Zur Kombination eignen sich marktübliche Melanogeneseinhibitoren wie z.B. Ascorbinsäure und deren Derivate, Aloesin, Niacinamide, Emblica, Elaginsäure, Licorice-Extrakt, Maulbeerbaumextrakt, Kojisäure, Süßholzwurzelextrakt, Rucinol, Hydrochinon, Azelainsäure, Arbutin, Magnesium-ascorbyl-phosphat oder dergleichen. Bevorzugte Beispiele von Verbindungen mit hautaufhellender Aktivität sind Hydrochinon, Niacinamid, Ascorbinsäure und Salze davon, Kojisäure, Arbutin, Aloesin, Azelainsäure, Elaginsäure oder Rucinol. Bevorzugte Beispiele von Extrakten mit hautaufhellender Aktivität sind Licorice-Extrakt, Maulbeerbaumextrakt oder Emblica.

Die erfindungsgemäßen Zubereitungen können darüber hinaus Anti-Aging-Wirkstoffe, Anti-Cellulite-Wirkstoffe oder übliche hautschonende oder hautpflegende Wirkstoffe enthalten. Hautschonende oder hautpflegende Wirkstoffe können prinzipiell alle dem Fachmann bekannten Wirkstoffe sein. Besonders bevorzugte Anti-Aging-Wirkstoffe sind Pyrimidincarbonsäuren, Aryloxime, Bioflavonoide, bioflavonoidhaltige Extrakte, Chromone oder Retinoide.

Geeignete Anti-Aging Wirkstoffe, insbesondere für hautpflegende Zubereitungen, sind vorzugsweise auch sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin-Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z.B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N-Acetylornithin, Trimethylamin-N-oxid, Di-myo-inositol-phosphat (DIP), cyclisches 2,3-Diphosphoglycerat (cDPG), 1,1- Diglycerin-Phosphat (DGP), ß-Mannosylglycerat (Firoin), ß-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen.

Additional können als Anti-Aging-Wirkstoffe Produkte der Firma Merck, wie z.B. 5,7-Dihydroxy-2-methyl-chromon, vermarktet unter dem Handelsnamen RonaCare^{®} Luremine, Ronacare^{®} Isoquercetin, Ronacare^{®} Tilirosid oder Ronacare^{®} Cyclopeptide 5 verwendet werden.

Bekannte Anti-Aging-Stoffe sind auch Chromone, wie beispielsweise in EP 1508327 beschrieben oder Retinoide, beispielsweise Retinol (Vitamin A), Retinsäure, Retinaldehyd oder auch synthetisch modifizierte Verbindungen von Vitamin A. Die beschriebenen Chromone und Retinoide sind gleichzeitig auch wirksame Anti-Cellulite-Wirkstoffe. Ein ebenfalls bekannter Anti-Cellulite-Wirkstoff ist Koffein.

Ein Gegenstand der Erfindung ist ebenfalls eine Zubereitung enthaltend mindestens eine Verbindung der Formel (I), wie zuvor beschrieben, und eine Selbstbräunungssubstanz, die einen Kontrastreduktionseffekt aufweist und einen ebenmäßigen Hautton erzielt. Ebenso Gegenstand der Erfindung ist die Verwendung von Verbindungen der Formel (I), wie beschrieben, in Kombination mit Selbstbräunungssubstanzen zur Kontrastreduktion und Erzielung eines ebenmäßigen Hauttones. Ein Kontrastreduktionsmittel ist demzufolge eine Substanz, die eine ungleichmäßige Hautfärbung reduziert, in dem sie den Kontrast zwischen stärkeren und weniger stark gefärbten Hautpartien herabsetzt. Dabei kann eine derartige untergleichmäßige Hautfärbung durch eine ungleichmäßige Pigmentierung und/oder eine unterschiedliche Verteilung der Hornhaut zustande kommen. Eine ungleichmäßige Pigmentierung ist in der Bevölkerung durchaus nicht unüblich und beruht auf einer unterschiedlich starken Melaninproduktion der Melanozyten oder einer unregelmäßigen Verteilung der Melanozyten in der Haut. Die Vereinigung von bräunenden Mischungen, die auf der Maillard-Reaktion oder Michael-Addition beruhen, mit melanogenesehemmenden Substanzen bewirkt, dass bereits hyperpigmentierte Hautareale ihre hohen Melaninkonzentrationen verlieren und sich der durch das Färbemittel an der Hautoberfläche erzeugte Farbton großflächig durchsetzt.

Eine Kontrastreduktion kann insbesondere durch Zubereitungen erzielt werden, in denen zumindest eine Verbindung nach Formel (I) zusätzlich mit einer Selbstbräunungssubstanz, vorzugsweise Dihydroxyaceton (DHA) und daraus abgeleitete Derivate, DHA rapid, DHA plus oder Erythrulose enthaltend, oder einer Mischung von Selbstbräunungssubstanzen, vorzugsweise DHA, DHA rapid, DHA plus und/oder Erythrulose enthaltend, zusammengebracht werden. Als vorteilhafte Selbstbräuner in einer Dihydroxyaceton enthaltenden Mischung oder Zubereitung können unter anderem eingesetzt werden: Glycerolaldehyd, Hydroxymethylglyoxal, γ-Dialdehyd, 6-Aldo-D-Fructose, Ninhydrin, 5-Hydroxy-1,4-naphtochinon (Juglon) oder 2-Hydroxy-1,4-naphtochinon (Lawson) oder eine Mischung der genannten Verbindungen. Besonders bevorzugt wird Erythrulose in der Dihydroxyaceton enthaltenden Mischung eingesetzt.

Die mindestens eine Verbindung der Formel (I) kann auch zusammen mit einer Mischung von Selbstbräunungssubstanzen enthaltend mindestens Dihydroxyaceton und einen weiteren Selbstbräuner ausgewählt aus der zuvor genannten Gruppe erfindungsgemäß verwendet werden. Beispielhaft besteht die erfindungsgemäß zu verwendende Mischung aus Dihydroxyaceton und mindestens einer weiteren Selbstbräunungssubstanz, wie zuvor beschrieben. Diese Mischung kann dann erfindungsgemäß mit mindestens einer Verbindung der Formel (I) kombiniert und in kosmetischen, dermatologischen oder pharmazeutischen Zubereitungen eingesetzt werden, wie nachfolgend beschrieben. Ganz besonders bevorzugt wird Dihydroxyaceton oder ein davon abgeleitetes Derivat ohne weitere Selbstbräunungssubstanzen eingesetzt.

In den beschriebenen Zubereitungen, die erfindungsgemäß mindestens eine Verbindung nach Formel (I) und einen Selbstbräuner enthalten, können weiterhin auch Farbpigmente enthalten sein, wobei der Schichtaufbau der Pigmente nicht limitiert ist. Vorzugsweise sollte das Farbpigment bei Einssatz von 0,5 bis 5 Gew.-% hautfarben oder bräunlich sein. Die Auswahl eines entsprechenden Pigments ist für den Fachmann geläufig.

Die beschriebenen Mittel oder Zubereitungen eignen sich besonders zur Verwendung bei der nicht-therapeutischen Aufhellung von Haut, Hemmung von Tyrosinase in-vitro und/oder zur Verwendung in der Prophylaxe, Therapie und/oder Verlaufskontrolle von Pigmentstörungen der Haut, in allen Fällen insbesondere bei Hyperpigmentierung, Sommersprossen, Altersflecken, Sonnenflecken sowie umweltbedingter Hautalterung. Dabei liegen sie in verschiedenen, für diese Anwendung üblicherweise verwendeten Darreichungsformen vor. Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole, Pflaster, Umschläge, Verbände und Sprays, insbesondere für die äußerliche Anwendung. Weitere Anwendungsformen sind z.B. Sticks, Shampoos und Duschbäder. Erfindungsgemäße kosmetische und dermatologische Zubereitungen können insbesondere eine wasserfreie Zubereitung, eine Lotion oder Emulsion, wie als Creme oder Milch, oder Mikroemulsion, jeweils vom Typ Wasser-in-Öl (W/O) oder vom Typ Öl-in-Wasser (O/W), eine multiple Emulsion, beispielsweise vom Typ Waser-in-Öl-in-Wasser (W/O/W) oder umgekehrt (O/W/O), Gele bzw. Lösungen (insbesondere ölig-alkoholische, ölig-wässrige oder wässrig-alkoholische Gele bzw. Lösungen), einen festen Stift, eine Salbe oder auch ein Aerosol darstellen. Zur Anwendung werden die erfindungsgemäßen kosmetischen und dermatologischen Zubereitungen in der für Kosmetika üblichen Weise auf die Haut in ausreichender Menge aufgebracht.

Eine Ausführungsform der Erfindung ist eine Emulsion, welche als Creme oder Milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält. Weitere besonders bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar. Eine besonders bevorzugte erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die ölig-alkoholischen Gele enthalten außerdem natürliches oder synthetisches Öl oder Wachs. Die festen Stifte bestehen vorzugsweise aus natürlichen oder synthetischen Wachsen und Ölen, Fettalkoholen, Fettsäuren, Fettsäureestern, Lanolin und anderen Fettkörpern. Ist eine Zubereitung als Aerosol konfektioniert, verwendet man vorzugsweise die üblichen Treibmittel, wie Alkane, Luft, Stickstoff, Distickstoffmonoxid, besonders bevorzugt Alkane oder Luft.

Die eine oder die mehreren Verbindungen der Formel (I), wie beschrieben, können in der üblichen Weise in kosmetische oder dermatologische Zubereitungen eingearbeitet werden. Die Zubereitung wird beispielsweise so gestaltet, dass sie für eine orale Gabe geeignet ist. Es ist auch vorteilhaft, Ectoine in verkapselter Form darzureichen, z. B. in Kollagenmatrices und anderen üblichen Verkapselungsmaterialien, wie z.B. als Cellulose- oder Chitinkapseln, in Gelatine bzw. Wachsmatrices oder liposomal verkapselt. Insbesondere Wachsmatrices wie sie in der DE-A-4308282 beschrieben sind, haben sich als günstig herausgestellt.

Die Zubereitungen können die genannten notwendigen oder optionalen Bestandteile oder Inhaltsstoffe umfassen oder enthalten, im Wesentlichen oder daraus bestehen. Alle Verbindungen oder Komponenten, die in den Zubereitungen verwendet werden können, sind entweder bekannt und käuflich erwerbbar oder können nach bekannten Verfahren synthetisiert werden. Der Zubereitung können beliebige übliche Trägerstoffe, Hilfsstoffe und gegebenenfalls weitere Wirkstoffe zugesetzt werden. Vorzuziehende Hilfsstoffe stammen aus der Gruppe der Konservierungsstoffe, Stabilisatoren, Lösungsvermittler, Färbemittel, d.h. Pigmente, Farbstoffe, Emulgatoren oder Geruchsverbesserer.

Salben, Pasten, Cremes und Gele können die üblichen Trägerstoffe enthalten, die für die topische Verabreichung geeignet sind, wie z.B. tierische und pflanzliche Fette, Wachse, Paraffine, Stärke, Traganth, Cellulosederivate, Polyethylenglykole, Silicone, Bentonite, Kieselsäure, Talkum und Titandioxid oder Gemische dieser Stoffe. Puder und Sprays können die üblichen Trägerstoffe enthalten, wie z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, wie z.B. Chlorfluorkohlenwasserstoffe, Propan/Butan oder Dimethylether, enthalten. Auch Druckluft ist vorteilhaft zu verwenden. Luft kann aber auch in drucklosen Dosiervorrichtungen, wie z.B. Pumpsprays, eingesetzt werden. Lösungen und Emulsionen können die üblichen Trägerstoffe wie Lösungsmittel, Lösungsvermittler und Emulgatoren, wie z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten; ein bevorzugter Lösungsvermittler generell ist 2-Isopropyl-5-methyl-cyclohexancarbonyl-D-Alaninmethylester. Suspensionen können die üblichen Trägerstoffe wie flüssige Verdünnungsmittel, wie z.B. Wasser, Ethanol oder Propylenglykol, Suspendiermittel, wie z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten. Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten. Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten. Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Zu den bevorzugten erfindungsgemäßen Zubereitungsformen gehören insbesondere Emulsionen. O/W-Emulsionen werden besonders bevorzugt. Emulsionen, W/O-Emulsionen und O/W-Emulsionen sind in üblicher Weise erhältlich. Erfindungsgemäße Emulsionen sind vorteilhaft und enthalten z.B. die genannten Fette, Öle, Wachse und anderen Fettkörper, sowie Wasser bzw. eine wässrige Phase, beispielsweise mit Lösemitteln oder hydrophilen Tensiden, und einen Emulgator, wie er üblicherweise für einen solchen Typ der Zubereitung verwendet wird.

Die Lipidphase kann vorteilhaft aus folgenden Substanzgruppen gewählt werden:
- Mineralöle, Mineralwachse;
- Öle, wie z.B. Triglyceride der Caprin- oder der Caprylsäure, ferner natürliche Öle, wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, wie z.B. Jojobaöl. Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr. Auch beliebige Abmischungen solcher Öl und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, wie z.B. Ethanol, Isopropanol, 1,2-Propandiol, Glycerin sowie insbesondere ein oder mehrere Verdickungsmittel, welches oder welche vorteilhaft gewählt werden können aus der Gruppe von Siliciumdioxid, Aluminiumsilikate, Polysaccharide bzw. deren Derivaten, wie z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Insbesondere werden Gemische der vorstehend genannten Lösemittel verwendet. Bei alkoholischen Lösemitteln kann Wasser ein weiterer Bestandteil sein.

In einer bevorzugten Ausführungsform enthalten die erfindungsgemäßen Zubereitungen hydrophile Tenside. Die hydrophilen Tenside werden bevorzugt gewählt aus der Gruppe der Alkylglucoside, der Acyllactylate, der Betaine sowie der Cocoamphoacetate.

Als Emulgatoren können beispielsweise die bekannten W/O- und O/W-Emulgatoren verwendet werden. Es ist vorteilhaft, weitere übliche Co-Emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen.

Es ist ferner von Vorteil, die Fettsäureethoxylate aus folgender Gruppe zu wählen: Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)-isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth14sulfat vorteilhaft verwendet werden. Als ethoxyliertes Cholesterinderivat kann vorteilhaft Polyethylenglycol(30)Cholesterylether verwendet werden. Auch Polyethylenglycol(25)-Sojasterol hat sich bewährt. Als ethoxylierte Triglyceride können vorteilhaft die Polyethylenglycol(60) Evening Primrose Glycerides verwendet werden (=Nachtkerze).

Weiterhin ist von Vorteil, die Polyethylenglycolglycerinfettsäureester aus der Gruppe von Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glyceryl-caprat/cprinat, Polyethylenglycol(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitan-monolaurat, Polyethylenglycol(20)sorbitanmonostearat, Polyethylenglycol(20)sorbitanmonoisostearat, Polyethylenglycol(20)sorbitanmonopalmitat und Polyethylenglycol(20)-sorbitanmonooleat zu wählen.

Als fakultative, dennoch erfindungsgemäß gegebenenfalls vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 bis 18 C-Atome, Diglycerinester gesättigter und/oder ungesättigter, verzweigter und/oder unverzweigter Alkancarbonsäuren einer Kettenlänge von 8 bis 24, insbesondere 12 bis 18 C-Atomen, Monoglycerinether gesättigter und/oder ungesättigter, verzweigter und/oder unverzweigter Alkohole einer Kettenlänge von 8 bis 24, insbesondere 12 bis 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat oder PEG-30-dipolyhydroxystearat.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Gegenstand der Erfindung ist auch ein Verfahren zum Herstellen und optional Herrichten einer erfindungsgemäßen Zubereitung mit den folgenden Schritten: (a) Vermischen mindestens einer Verbindung der Formel (I), Teilformel (IA), (IB), (IC) und/oder ihrer physiologisch unbedenklichen Salze, Tautomere, Stereoisomere und/oder Solvate, einschließlich deren Mischungen in allen Verhältnissen, mit mindestens einem weiteren Wirkstoff und mindestens einem für topische Anwendungen geeigneten Träger und optional mit physiologisch unbedenklichen Hilfsstoffen und/oder Füllstoffen, und optional (b) augenfälliges Herrichten der Verbindungen der Formel (I). Die erfindungsgemäßen Zubereitungen können dabei mit Hilfe von Techniken hergestellt werden, die dem Fachmann wohl bekannt sind. Das Vermischen kann ein Lösen, Emulgieren oder Dispergieren der mindestens einen Verbindung gemäß Formel (I) oder Teilformel davon, wie zuvor beschrieben, in dem Träger zur Folge haben. Die vorherige Lehre der Erfindung und deren Ausführungsformen betreffend die Verbindungen der Formel (I) und Zubereitungen damit ist gültig und ohne Einschränkungen auf die Herstellung und optional Herrichtung der Zubereitung anwendbar, sofern es sinnvoll erscheint.

Noch ein Gegenstand der Erfindung betrifft Verbindungen der Formel (I) 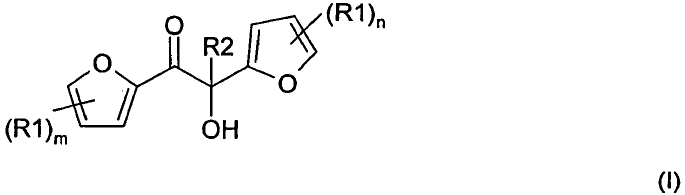 worin
- R1: A, Cyc, OH oder Ar,
- R2: Y, Cyc, OH oder Ar,
- Y: H oder Alk,
- A: unverzweigtes oder verzweigtes Alkyl mit 5-18 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- oder -CΞC-Gruppe ersetzt sein können,
- Alk: unverzweigtes oder verzweigtes Alkyl mit 1-12 C-Atomen,
- Cyc: zyklisches Alkyl mit 3-8 C-Atomen, wobei unabhängig voneinander 1-3 H-Atome durch F und/oder unabhängig voneinander eine oder zwei benachbarte CH₂-Gruppen durch -O-, -N(Alk)- oder eine -CH=CH- Gruppe ersetzt sein können,
- Ar: ein-, zwei- oder dreikerniges Aryl mit 5 bis 14 C-Atomen, das unsubstituiert oder ein-, zwei- oder dreifach durch F, Alk oder -(CY₂)ₚ-OY substituiert sein kann,und
- m, p: unabhängig voneinander 0, 1 oder 2 bedeuten,
unter der Maßgabe, dass Y und Ar für R2 ausgeschlossen sind, wenn m und n gleichzeitig 0 bedeuten,
und/oder ihre physiologisch unbedenklichen Salze, Tautomere, Stereoisomere und/oder Solvate, einschließlich deren Mischungen in allen Verhältnissen.

Eine bevorzugte Ausgestaltung der Erfindung betrifft Verbindungen der Teilformel (IC) 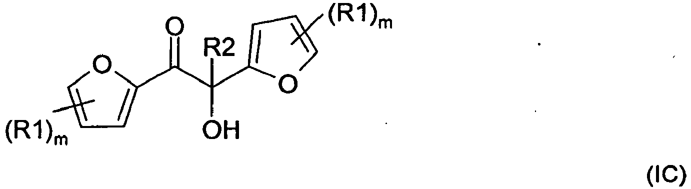 worin
- R1: A, Cyc, OH oder Ar,
- R2: Y, Cyc, OH oder Ar,
- Y: H oder Alk,
- A: unverzweigtes oder verzweigtes Alkyl mit 5-8 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- oder -CΞC-Gruppe ersetzt sein können,
- Alk: unverzweigtes oder verzweigtes Alkyl mit 1-4 C-Atomen,
- Cyc: zyklisches Alkyl mit 3-8 C-Atomen, wobei unabhängig voneinander 1-3 H-Atome durch F und/oder unabhängig voneinander eine oder zwei benachbarte CH₂-Gruppen durch -O-, -N(Alk)- oder eine -CH=CH- Gruppe ersetzt sein können,
- Ar: ein-, zwei- oder dreikerniges Aryl mit 5 bis 14 C-Atomen, das unsubstituiert oder ein-, zwei- oder dreifach durch F, Alk oder -(CY₂)ₚ-OY substituiert sein kann, und
- m, p: unabhängig voneinander 0, 1 oder 2 bedeuten,
unter der Maßgabe, dass Y und Ar für R2 ausgeschlossen sind, wenn m 0 bedeutet, und/oder ihre physiologisch unbedenklichen Salze, Tautomere, Stereoisomere und/oder Solvate, einschließlich deren Mischungen in allen Verhältnissen.

In einer besonders bevorzugten Ausgestaltung der Erfindung werden Verbindungen der Teilformel (IC) bereitgestellt, worin
- R1: A, Cyc oder Ar,
- R2: Cyc,
- Y: H oder Alk,
- A: unverzweigtes oder verzweigtes Alkyl mit 5-8 C-Atomen, wobei unabhängig voneinander 1-3 H-Atome durch F und/oder unabhängig voneinander eine oder zwei benachbarte CH₂-Gruppen durch eine -CH=CH- oder -CΞC- Gruppe ersetzt sein können,
- Alk: unverzweigtes oder verzweigtes Alkyl mit 1-4 C-Atomen,
- Cyc: zyklisches Alkyl mit 3-8 C-Atomen, wobei unabhängig voneinander 1-3 H-Atome durch F und/oder unabhängig voneinander eine oder zwei benachbarte CH₂-Gruppen durch eine -CH=CH- Gruppe ersetzt sein können,
- Ar: ein-, zwei- oder dreikerniges Aryl mit 5 bis 14 C-Atomen, das unsubstituiert oder ein- oder zweifach durch F oder OY substituiert sein kann, und
- m: 0, 1 oder 2 bedeuten,
und/oder ihre physiologisch unbedenklichen Salze, Tautomere, Stereoisomere und/oder Solvate, einschließlich deren Mischungen in allen Verhältnissen.

Noch ein weiterer Gegenstand der Erfindung betrifft ein Verfahren zum Herstellen von Verbindungen der Teilformel (IC) und/oder ihrer Salze, Tautomere, Stereoisomere und/oder Solvate, einschließlich deren Mischungen in allen Verhältnissen, mit den folgenden Schritten:
(a) Benzoin-Addition von Furfural-Derivaten der Formel (II) 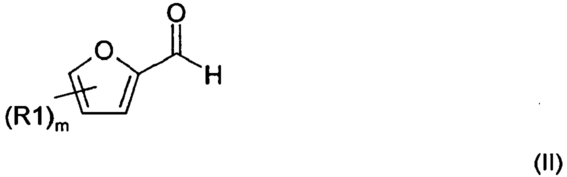 worin R1 und m die oben angegebene Bedeutung aufweisen,
   unter Erhalt von Verbindungen der Teilformel (IC) 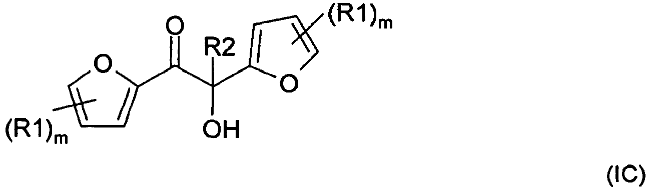 worin R1, R2 und m die oben angegebene Bedeutung aufweisen,
   und optional
(b) Umwandeln einer Base oder Säure der Verbindungen der Teilformel (IC) in eines ihrer Salze.

Die Verbindungen der Formel (I) sowie Teilformel (IC) eignen sich insbesondere zur Verwendung bei der nicht-therapeutischen Aufhellung von Haut, Hemmung von Tyrosinase in-vitro, und/oder zur Verwendung in der Prophylaxe, Therapie und/oder Verlaufskontrolle von Pigmentstörungen der Haut. Diese Verbindungen können insbesondere auch mit Selbstbräunungssubstanzen kombiniert werden, die auf dem Prinzip der Erzeugung von Farbpigmenten durch nicht-enzymatische Bräunungsreaktion der Selbstbräunungssubstanzen mit keratinhaltigen Matrizes beruhen, so dass ein ebenmäßigerer Hautfarbton erzielt wird. Im Übrigen ist die vorherige Lehre der Erfindung und deren Ausführungsformen betreffend die Verwendung von Verbindungen der Formel (I) und deren Herstellung gültig und ohne Einschränkungen auf die Verbindungen der Teilformel (IC) sowie deren Herstellung und Verwendung anwendbar, sofern es sinnvoll erscheint.

Eine andere Ausgestaltung der vorliegenden Erfindung bezieht sich auf ein Arzneimittel, umfassend mindestens eine Verbindung der Formel (I) bzw. Teilformel (IC) und/oder ihre physiologisch unbedenklichen Salze, Tautomere, Stereoisomere und/oder Solvate, einschließlich deren Mischungen in allen Verhältnissen. Noch eine andere Ausgestaltung der Erfindung ist eine pharmazeutische Zusammensetzung umfassend als Wirkstoff eine wirksame Menge von mindestens einer Verbindung der Formel (IC) und/oder ihrer physiologisch unbedenklichen Salze, Tautomere und/oder Stereoisomere, einschließlich deren Mischungen in allen Verhältnissen, zusammen mit pharmazeutisch verträglichen Hilfsstoffen. Für beide Ausgestaltungen ist eine Verbindung der Teilformel (IC) bevorzugt.

Ein "Arzneimittel", "Medikament" sowie eine "pharmazeutische Zusammensetzung" oder pharmazeutische Formulierung" ist hierbei jedes Mittel, welches in der Prophylaxe, Therapie, Verlaufskontrolle oder Nachbehandlung von Patienten eingesetzt werden kann, die zumindest zeitweise eine pathogene Modifikation des Gesamtzustandes bzw. des Zustandes einzelner Teile des Patientenorganismus zeigen, vorzugsweise infolge von Pigmentstörungen der Haut.

Um die protektive oder therapeutische Wirkung der erfindungsgemäßen Verbindungen zu erhöhen, können pharmazeutisch verträgliche Adjuvantien zugesetzt werden. Im Sinne der Erfindung ist jede Substanz, die mit den Verbindungen gemäß der Erfindung eine Wirkung ermöglicht, verstärkt oder modifiziert, ein "Adjuvans". Bekannte Adjuvantien sind z.B. Aluminiumverbindungen, wie z.B. Aluminiumhydroxid oder Aluminiumphosphat, Saponine, wie z.B. QS 21, Muramyldipeptid oder Muramyltripeptid, Proteine, wie z.B. Gammainterferon oder TNF, MF 59, Phosphatdibylcholin, Squalen oder Polyole. Des Weiteren kann DNA, die ein Protein mit Adjuvanseffekt kodiert, parallel oder in einem Konstrukt appliziert werden.

Das Einbringen des pharmazeutischen Mittels in eine. Zelle respektive einen Organismus kann erfindungsgemäß auf jede Art und Weise geschehen, die es ermöglicht, dass Tyrosinase mit den in der Zusammensetzung enthaltenen Verbindungen in Kontakt gebracht und infolgedessen eine Antwort induziert wird. Das pharmazeutische Mittel der vorliegenden Erfindung kann oral, topisch, transdermal, transmucosal, transurethal, vaginal, rektal, pulmonal, enteral und/oder parenteral angewendet werden, vorzugsweise topisch bzw. transdermal. Die gewählte Art der Verabreichung richtet sich nach der Indikation, der zu verabreichenden Dosis, Individuums-spezifischen Parametern etc. Insbesondere ermöglichen die verschiedenen Arten der Verabreichung eine ortsspezifische Therapie, die Nebenwirkungen minimiert und die Wirkstoffdosis verringert.

Die Darreichungsformen des pharmazeutischen Mittels werden mit den üblichen festen oder flüssigen Trägerstoffen und/oder Verdünnungsmitteln und den üblicherweise eingesetzten Hilfsstoffen entsprechend der gewünschten Applikationsart in einer geeigneten Dosierung und in an sich bekannter Weise hergestellt. Grundsätzlich können also pharmazeutisch annehmbare und dem Fachkundigen bekannte Exzipienten einen Teil des erfindungsgemäßen pharmazeutischen Mittels bilden, wobei die Menge des Exzipientenmaterials, die mit dem Wirkstoff kombiniert wird, um eine Einzeldosierung herzustellen, in Abhängigkeit vom zu behandelnden Individuum und der Art der Verabreichung variiert. Diese pharmazeutisch verträglichen Zusätze umfassen Salze, Puffer, Füllstoffe, Stabilisatoren, Komplexbildner, Antioxidantien, Lösungsmittel, Bindemittel, Gleitmittel, Tablettenüberzüge, Geschmacksstoffe, Farbstoffe, Konservierungsmittel, Stellmittel und dergleichen. Beispiele für solche Exzipienten sind Wasser, pflanzliche Öle, Benzylalkohole, Alkylenglycol, Polyethylenglycol, Glycerintriacetat, Gelatine, Kohlenhydrate, wie z.B. Laktose oder Stärke, Magnesiumstearat, Talk und Vaseline.

Die pharmazeutische Formulierung kann als Tablette, Filmtablette, Dragee, Lutschtablette, Kapsel, Pille, Pulver, Granulat, Sirup, Saft, Tropfen, Lösung, Dispersion, Suspension, Suppositor, Emulsion, Implantat, Creme, Gel, Salbe, Paste, Lotion, Serum, Öl, Spray, Aerosol, Kleber, Pflaster oder Verband vorliegen. Als orale Darreichungsform werden vorzugsweise Tabletten, Filmtabletten, Dragees, Lutschtabletten, Kapseln, Pillen, Pulver, Granulate, Sirupe, Säfte, Tropfen, Lösungen, Dispersionen oder Suspensionen - auch als Depotform - zubereitet. Des Weiteren sind parenterale Arzneiformen, wie z.B. Suppositorien, Suspensionen, Emulsionen, Implantate oder Lösungen, in Betracht zu ziehen, vorzugsweise ölige oder wässrige Lösungen. Zur topischen Applikation wird der Arzneimittelwirkstoff mit mindestens einem pharmazeutisch annehmbaren Trägerstoff, wie z.B. mikrokristalliner Cellulose, und ggf. weiteren Hilfsstoffen, wie z.B. Feuchtigkeitsspendern, zu auf die Haut auftragbaren festen Formulierungen, wie z.B. Cremes, Gelen, Salben, Pasten, Pulver oder Emulsionen bzw. zu auf die Haut auftragbaren flüssigen Formulierungen, wie z.B. Lösungen, Suspensionen, Lotionen, Seren, Ölen, Sprays oder Aerosole in üblicher Weise formuliert. Vorzugsweise liegt das pharmazeutische Mittel zur topischen Applikation vor. Das pharmazeutische Mittel kann auch als feste Zusammensetzung, beispielsweise im lyophilisierten Zustand vorliegen, und dann durch Zugabe eines auflösenden Mittels, wie z.B. destilliertes Wasser, vor der Verwendung bereitet werden. Die Grundlagen der Herstellung von Lyophilisaten sind dem Fachmann vertraut.

Die Konzentration der aktiven Verbindung in der Formulierung kann 0,01 bis 100 Gewichtsprozente betragen. Entscheidend ist, dass die pharmazeutische Zusammensetzung als Wirkstoff eine effektive Menge der Verbindung zusammen mit den pharmazeutisch verträglichen Hilfsstoffen umfasst. Die Begriffe "effektive Menge" oder "wirksame Dosis" werden hierin austauschbar verwendet und bezeichnen eine Menge des pharmazeutischen Wirkstoffs, die eine prophylaktisch oder therapeutisch relevante Wirkung auf eine Krankheit oder pathologische Veränderung in Zelle, Gewebe, Organ oder Säuger hat. Eine "prophylaktische Wirkung" verhindert das Ausbrechen einer Krankheit und umfasst auch die Erhöhung der normalen physiologischen Funktion. Eine Prophylaxe ist insbesondere ratsam, wenn ein Individuum Veranlagungen für den Ausbruch der vorgenannten Krankheiten aufweist, wie z.B. eine familiäre Vorbelastung, einen Gendefekt oder eine kürzlich überstandene Krankheit. Eine therapeutisch relevante Wirkung" befreit teilweise oder vollständig von einem, mehreren oder allen Krankheitssymptomen oder führt zur teilweisen oder vollständigen Rückkehr eines, mehrerer oder aller physiologischer oder biochemischer Parameter, die mit der Krankheit oder pathologischen Veränderung in Zusammenhang stehen oder ursächlich dafür sind, in den Normalzustand. Auch wird die Verlaufskontrolle als Art der therapeutischen Behandlung verstanden, wenn die Verbindungen in bestimmten Intervallen verabreicht werden, z.B. um die Symptome einer Krankheit vollständig zu beseitigen. Die jeweilige Dosis bzw. der Dosisbereich für die Gabe der erfindungsgemäßen Verbindungen ist groß genug, um den gewünschten prophylaktischen oder therapeutischen Effekt der Induktion einer biologischen oder medizinischen Antwort zu erreichen. Im Allgemeinen wird die Dosis mit dem Alter, der Konstitution und dem Geschlecht des Patienten variieren sowie die Schwere der Erkrankung berücksichtigen. Es versteht sich, dass die spezifische Dosis, Häufigkeit und Dauer der Verabreichung darüber hinaus von einer Vielzahl an Faktoren abhängen, wie z.B. der Zielsteuerungs- und Bindungsfähigkeit der Verbindungen, Ernährungsgewohnheiten des zu behandelnden Individuums, Art der Verabreichung, Ausscheidungsrate und Kombination mit anderen Medikamenten. Die individuelle Dosis kann sowohl in Bezug auf die primäre Erkrankung als auch in Bezug auf das Eintreten eventueller Komplikationen eingestellt werden. Die exakte Dosis ist durch einen Fachmann mit bekannten Mitteln und Methoden feststellbar.

In einer Ausführungsform der Erfindung werden die Verbindungen in einer Dosis von 0,01 mg bis 1 g pro Dosierungseinheit verabreicht, vorzugsweise zwischen 1 bis 700 mg, besonders bevorzugt 5 bis 100 mg. Die tägliche Dosierung liegt insbesondere zwischen 0,02 und 100 mg/kg Körpergewicht.

Zur Unterstützung der medizinischen Wirkung kann die pharmazeutische Zusammensetzung in einer Ausgestaltung der Erfindung auch einen oder mehrere weitere Wirkstoffe umfassen, wobei eine gleichzeitige oder aufeinander folgende Verabreichung denkbar ist. Die therapeutische Wirkung der erfindungsgemäßen pharmazeutischen Zusammensetzung kann beispielsweise darin bestehen, dass durch die Hemmung der Tyrosinase als erwünschter Nebeneffekt bestimmte Hautaufheller besser wirken oder durch die Verminderung der Dosis die Anzahl der Nebenwirkungen dieser Medikamente reduziert wird. Die vorherige Lehre der Erfindung und deren Ausführungsformen betreffend die Kombination von Verbindungen der Formel (I) mit weiteren Wirkstoffen sowie solche Zubereitungen ist gültig und ohne Einschränkungen auf Kombinationspräparate mit den Verbindungen der Teilformel (IC) sowie deren Zubereitungen anwendbar, sofern es sinnvoll erscheint.

Die Erfindung kann auch als Kit praktiziert werden, der die erfindungsgemäßen Verbindungen enthält. Das Kit besteht aus getrennten Packungen von (a) einer wirksamen Menge einer Verbindung der Teilformel (IC) und/oder ihrer physiologisch unbedenklichen Salze, Tautomere und/oder Stereoisomere, einschließlich deren Mischungen in allen Verhältnissen, und (b) einer wirksamen Menge eines weiteren Wirkstoffs. Das Kit enthält geeignete Behälter, wie z.B. Schachteln oder Kartons, individuelle Flaschen, Beutel oder Ampullen. Das Kit kann z.B. separate Ampullen enthalten, in denen jeweils eine wirksame Menge an einer Verbindung der Teilformel (IC) und/oder ihrer pharmazeutisch verwendbaren Salze, Tautomere und/oder Stereoisomere, einschließlich deren Mischungen in allen Verhältnissen, und einer wirksamen Menge eines weiteren Arzneimittelwirkstoffs gelöst oder in lyophilisierter Form vorliegt. Das Kit der Erfindung kann auch einen Artikel beinhalten, der schriftliche Anweisungen enthält oder den Anwender auf schriftliche Anweisungen hinweist, die den Umgang mit den Verbindungen der Erfindung erläutern.

Im Rahmen der hier vorgestellten Erfindung wurden erstmals neuartige 2,2'-Furoin-Derivate der Formel (I) bereitgestellt. Die erfindungsgemäßen Verbindungen steuern affin und/oder selektiv Tyrosinase an. Die Verbindungen der Formel (I), deren Teilformeln und deren Derivate zeichnen sich durch eine hohe Spezifität und Stabilität, niedrige Herstellungskosten und leichte Handhabung aus. Auf diesen Eigenschaften basieren eine reproduzierbaren Wirkungsweise, einschließlich des Fehlens von Kreuzreaktivitäten, und die verlässliche und sichere Wechselwirkung mit den entsprechenden Zielstrukturen. Die Erfindung beinhaltet auch die Verwendung der vorliegenden 2,2'-Furoin-Derivate der Formel (I) zur Hemmung, Regulation und/oder Modulation der Signalkaskade von Tyrosinase und bietet damit neuartige Werkzeuge für Forschung und/oder Diagnostik.

Pharmazeutische Zusammensetzungen, die die genannten Verbindungen enthalten, und der Einsatz dieser Verbindungen zur Behandlung Tyrosinase-vermittelter Störungen sind ein vielversprechenden Ansatz, um bei Mensch und Tier eine direkte und unmittelbare Linderung von Symptomen zu erreichen. Dies ist besonders für die wirksame Behandlung von Pigmentstörungen vorteilhaft, entweder als Monotherapie oder in Kombination mit anderen hautaufhellenden Therapien. Die Kontrolle der Hautpigmentierung ist ebenso ein zentrales Thema moderner kosmetischer Produkte. Ob dabei ein möglichst bleiches oder eher ein gebräuntes Erscheinungsbild bevorzugt wird, ist entscheidend von kulturellen Faktoren abhängig. Produkte, die der Hautaufhellung dienen, sind beispielsweise im asiatischen Kulturraum von besonderem Interesse, wo ein heller Hautton mit gehobener sozialer Stellung und wirtschaftlichem Reichtum verknüpft wird.

Aufgrund der starken und selektiven Hemmung von Tyrosinase, die über die Melanin-Synthese den Hautton regelt, können die Verbindungen der Formel (I) erfindungsgemäß bei erheblich niedriger Konzentration verabreicht werden, während sie im Vergleich mit den weniger potenten hautaufhellenden Substanzen des Stands der Technik eine ähnliche oder sogar überlegene biologische Wirksamkeit erzielen. Sie zeigen vorteilhaft eine hohe und lang anhaltende Aktivität bezüglich ihrer Wirkung als hautaufhellende Wirkstoffe. Die Anwendung geht auch mit verringerten oder keinen medizinischen Nebenwirkungen einher, indem insbesondere keine Hautirritation auftreten. Die erfindungsgemäßen Wirkstoffe zeichnen sich dabei nicht nur durch eine verbesserte Wirksamkeit, sondern auch durch Anwendungssicherheit und gute Formulierbarkeit aus. So sind sie leicht in Zubereitungen einzuarbeiten und besitzen eine erhöhte Stabilität in den Zubereitungen.

Auch ohne weitere Ausführungen wird davon ausgegangen, dass ein Fachmann die obige Beschreibung in weitestem Umfang nutzen kann. Sämtliche genannte sowie weitere Bestandteile bzw. Komponenten sind dem Fachmann geläufig und können in Routineversuchen eine spezielle Ausgestaltung für die erfindungsgemäße Lehre erfahren. Sämtliche in der Beschreibung zitierten Dokumente sollen hiermit in ihrer Gesamtheit in die Offenbarung der vorliegenden Erfindung als Referenz einbezogen werden.

Die bevorzugten Ausführungsformen und Beispiele sind lediglich als beschreibende, keinesfalls als in irgendeiner Weise limitierende Offenbarung aufzufassen. Es versteht sich folglich, dass diese Erfindung nicht auf die spezifischen Verbindungen, pharmazeutischen Zusammensetzungen, Verwendungen und Verfahren beschränkt ist, wie sie hierin beschrieben sind, da solche Dinge variieren können. Es versteht sich des Weiteren, dass die vorliegend verwendete Terminologie ausschließlich dem Zweck der Beschreibung besonderer Ausführungsformen dient und nicht den Schutzumfang der Erfindung einschränken soll. Wie vorliegend in der Spezifikation einschließlich der anhängigen Ansprüche verwendet, schließen Wortformen im Singular, wie z. B. "ein", "nein", "einer", "der" oder "das" die Entsprechung im Plural ein, sofern der Kontext nicht eindeutig etwas anderes vorgibt. Beispielsweise enthält der Bezug auf "eine Verbindung" eine einzelne Verbindung oder mehrere Verbindungen, die wiederum identisch oder verschieden sein können, oder der Bezug auf "ein Verfahren" schließt äquivalente Schritte und Verfahren ein, die dem Fachmann bekannt sind.

Im Folgenden wird die Erfindung anhand nicht limitierender Beispiele für konkrete Ausführungsformen näher erläutert. Die Beispiele sind insbesondere dahingehend zu interpretieren, dass sie nicht auf die konkret veranschaulichten Merkmalskombinationen beschränkt sind, sondern die beispielhaften Merkmale wiederum frei kombiniert oder in Alleinstellung verwendet werden können, solange die Aufgabe der Erfindung gelöst wird. Die Gewichtsprozentverhältnisse der einzelnen Inhaltsstoffe in den Zubereitungen der Beispiele gehören ausdrücklich zur Offenbarung der Beschreibung und können daher als Merkmale herangezogen werden.

### BEISPIEL 1: Synthese von 2,2'-Furoin

Die Synthese erfindungsgemäßer Verbindungen ist aus entsprechend unsubstituierten oder substituierten Furfural-Derivaten mittels Benzoin-Kondensation möglich. Diese Reaktion ist vielfältig in der chemischen Literatur beschrieben worden. Im konkreten Fall kann die Synthese z.B. so erfolgen, wie sie in DE-A-19704273 beschrieben ist:

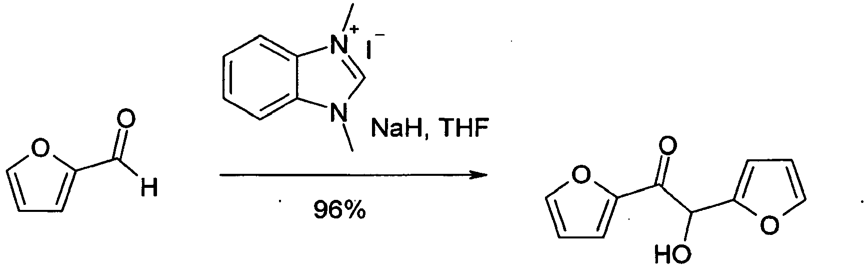

### BEISPIEL 2: Tyrosinase Assay

Die Wirkung der Verbindungen mit der Formel (I) als Hautaufheller wird durch ihre Fähigkeit geprüft, das Enzym Tyrosinase zu hemmen und damit die Melanin-Synthese zu unterbinden. Die hemmende Wirkung der Verbindungen der Formel (I) oder deren Teilformeln gegenüber Tyrosinase wird unter Verwendung von Tyrosinase aus Pilzen und L-DOPA als Substrat bewertet. Die Verbindungen und L-Dopa werden für 10 Minuten bei 25°C in Phosphatpuffer (pH 6.8) vorinkubiert und anschließend wird Tyrosinase aus Pilzen (16 U) (Fluka) zugegeben. Die optische Dichte der Proben wird bei 470 nm gegen eine Negativkontrolle (ohne Wirkstoff) gemessen. Kojisäure wird als Tyrosinase-Referenz, d.h. positive Kontrolle, mit getestet.

### BEISPIEL 3: B16 V Maus-Melanomzellen-Tests

B16V Maus-Melanomzellen (Hersteller: DSMZ; Artikel-Nr.: ACC370) werden in RPMI-Medium (Invitrogen, Artikel-Nr.: 31870), dem zusätzlich noch 10 % FBS (Fetal Bovine Serum; Invitrogen, Artikel-Nr.: 10499044), 2 mM L-Glutamin (Invitrogen, Artikel-Nr: 25030) und 1 mM Natriumpyruvat (Invitrogen, Artikel-Nr.: 11360) hinzugefügt werden, überführt und 72 h bei 37°C und 5 % CO₂ inkubiert. Das Medium wird abgetrennt und die Zellen werden einmal mit 10 mL DPBS (Dulbecco's Phosphate-Buffered Salines; Invitrogen, Artikel-Nr.: 14190) gewaschen und das Medium anschließend abgesaugt. Es wird 1 mL HyQtase- Cell Detachment Solution (Hyclone, Artikel-Nr.: SV30030.01) auf die Zellen gegeben. Die Flasche wird mehrfach geschwenkt und die HyQtase- Cell Detachment Solution anschließend durch Absaugen entfernt. Dann werden die Zellen für 5 min im Inkubator bei 37°C und 5 % CO₂ inkubiert. Die Zellen werden in dem modifizierten RPMI-Medium (s.o.) aufgenommen und die Zellzahl bestimmt. Dazu werden die Zellen mit Trypanblau angefärbt und in einer Neubauer-Zählkammer ausgezählt. Anschließend werden die Zellen erneut im modifizierten RPMI-Medium (s.o.) in einer definierten Zellzahl von 80000 Zellen pro Well (6 Well Clear Plate, TCT, PS (Nunc)) ausgesät.

Die Zellen werden 24 h bei 37°C und 5 % CO₂ inkubiert, danach das Medium entfernt. Anschließend werden 1980 µL der Substanzverdünnung zugegeben. Für diese Substanzverdünnung wird 2,2'-Furoin in DMSO gelöst und anschließend über einen Sterilfilter (0.2µm, Millipore, Artikel Nr. SLLG013SL) filtriert. Die Lösung wird dann mit dem modifizierten RPMI-Medium (s.o., jedoch beträgt in diesem Fall der FBS-Gehalt nur 5. %) so verdünnt, dass die Endkonzentration der 2,2'-Furoin-Verdünnung 0,1 mM bzw. 0,05 mM beträgt. Dann werden 20 µL einer alpha-MSH Lösung (alpha-melanocyte stimulating hormone, DMSO, Sigma, Artikel-Nr.: D2650) zugegeben, so dass die alpha-MSH Konzentration im Well bei 10⁻⁸ M liegt. Anschließend wird erneut 24 h bei 37°C und 5 % CO₂ inkubiert. Der in diesem Abschnitt beschriebene Vorgang wird insgesamt noch zweimal wiederholt.

Nach der letzten Inkubationsperiode wird das Medium abgesaugt und die Zellen mit 1000 µL DPBS (Invitrogen, Artikel-Nr.: 14190) gewaschen. Das Medium wird erneut abgesaugt. Es werden 250 µL HyQtase- Cell Detachment Solution (Hyclone, Artikel-Nr.: SV30030.01) auf die Zellen gegeben. Die 6 Well-Platte wird mehrfach geschwenkt und die HyQtase- Cell Detachment Solution anschließend durch Absaugen entfernt. Dann werden die Zellen für 5 min im Inkubator bei 37°C und 5 % CO₂ inkubiert. Die Zellen werden in 1,5 mL DPBS (Invitrogen, Artikel-Nr.: 14190) aufgenommen und in ein Cup (SARSTEDT, Ref. 72.692.005) überführt. Anschließend wird die Zellzahl bestimmt. Dazu werden die Zellen mit Trypanblau angefärbt und in einer Neubauer-Zählkammer ausgezählt. Die Zellen bei 3500 g 1 min zentrifugiert. Die erhaltenen Pellets werden photographiert und anschließend der Überstand abgesaugt. Die Pellets werden in 1 mL 1 N NaOH 1 h bei 80°C aufgelöst und dann auf RT abgekühlt. Anschließend werden pro Cup viermal je 200 µL (als Vierfachbestimmung) in eine 96 Well Platte (VWR, Artikel Nr.: 4100636981) pipettiert und die Absorption bei 405 nm Wellenlänge ermittelt (Safire, Tecan). Mittels einer Eichgeraden kann so der Gehalt an Melanin bestimmt werden.

**Tabelle 1: Hautaufhellender Effekt verschiedener Wirkstoffe im Vergleich.**

| Wirkstoff | Relativer Melaningehalt in % |
|---|---|
| DMSO (Lösemittel-Blindwert) | 100 |
| DMSO + alpha-MSH | 235 |
| Kojisäure (1 mM) + alpha-MSH | 96 |
| 2,2'-Furoin (0,1 mM) + alpha-MSH | 63,1 |
| 2,2'-Furoin (0,05 mM) + alpha-MSH | 88,0 |

Es kann im Vergleich zu im Stand der Technik bekannten hautaufhellenden Substanzen eine deutliche Verbesserung gezeigt werden, da mit erheblich niedrigeren Konzentrationen ein Effekt erzielt wird (Tabelle 1).

### BEISPIEL 4: Melanogenese-Assay mit humanen Primärzellen

Eine Ampulle Normaler Humaner Epidermaler Melanozyten (NHEM, Promocell) wird aufgetaut und je nach der Anzahl ihrer Verdopplungen in weitere Flaschen verteilt. Nach 2 bis 3 Wochen werden zwischen 750000 und einer Millionen Zellen in T25-Flaschen mit 5 ml Vollmedium überführt. Nach 24 Stunden wird das Medium entfernt und durch entsprechende Substanzverdünnungen bzw. Kontrollen ersetzt. Die Gesamtdauer der Inkubation der verschiedenen Prüfsubstanzen auf den Zellen beträgt 10 Tage. Während dieser 10 Tage werden alle 2-3 Tage die Substanzverdünnungen bzw. Kontrollen erneuert. Nach 10 Tagen werden die Melanozyten geerntet. Dabei wird 500 µl Trypsin/EDTA auf die Zellen gegeben und sofort wieder abgesaugt. Die Zellen werden anschließend in Medium aufgenommen, gezählt und abzentrifugiert. Der Überstand wird abgezogen, das Pellet mit DPBS gewaschen und wieder zentrifugiert. Von diesem Pellet wird ein Foto aufgenommen. Das Pellet wird nun in 1 ml NaOH (1 M) aufgenommen und eine Stunde bei 80°C im Schüttler inkubiert. Die auf Raumtemperatur abgekühlte Lösung wird in eine 96 Well Platte pipettiert (4 x 200 µl) und die Absorption bei 405 nm gemessen. Die gemessenen Werte werden anschließend prozentual gegen die Kontrolle aufgetragen.

**Tabelle 2: Hautaufhellender Effekt verschiedener Wirkstoffe im Vergleich.**

| Wirkstoff | Relativer Melaningehalt in % |
|---|---|
| DMSO | 100 |
| 2,2'-Furoin | 72,9 |

Es kann im Vergleich zu im Stand der Technik bekannten hautaufhellenden Substanzen eine deutliche Verbesserung gezeigt werden, da mit erheblich niedrigeren Konzentrationen ein Effekt erzielt wird (Tabelle 2).

### BEISPIEL 5: Zubereitungen

Im Folgenden werden beispielhaft Rezepturen für kosmetische Zubereitungen angegeben, die Verbindung nach Beispiel 1 enthalten (Tabellen 3-5). Im Übrigen sind die INCI-Bezeichnungen der handelsüblichen Verbindungen angegeben. UV-Pearl, OMC steht für die Zubereitung mit der INCI-Bezeichnung: Water (für EU: Aqua), Ethylhexyl Methoxycinnamate, Silica, PVP, Chlorphenesin, BHT; diese Zubereitung ist im Handel unter der Bezeichnung Eusolex^{®} UV Pearl™ OMC von der Merck KGaA, Darmstadt erhältlich. Die anderen in den Tabellen angegebenen UV-Pearl sind jeweils analog zusammengesetzt, wobei OMC gegen die angegebenen UV-Filter ausgetauscht wurde.

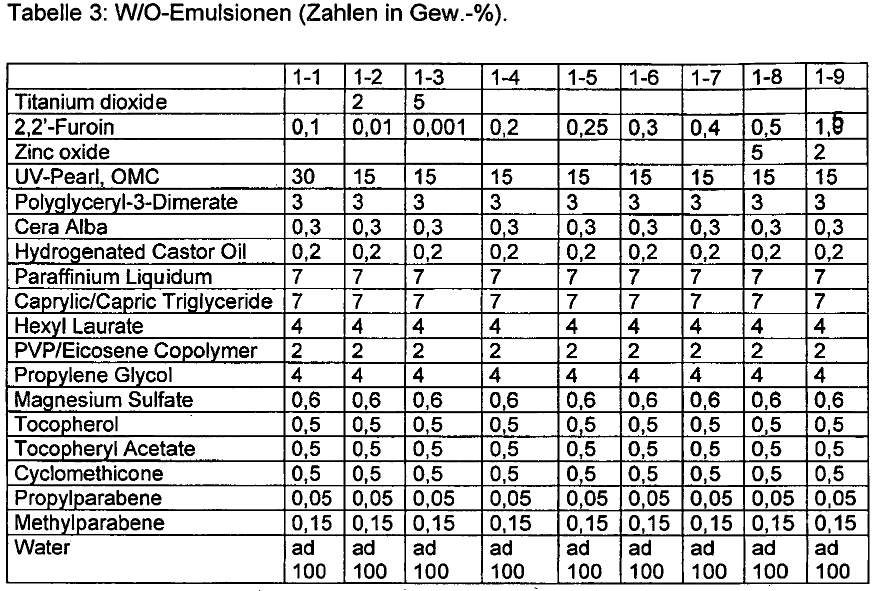

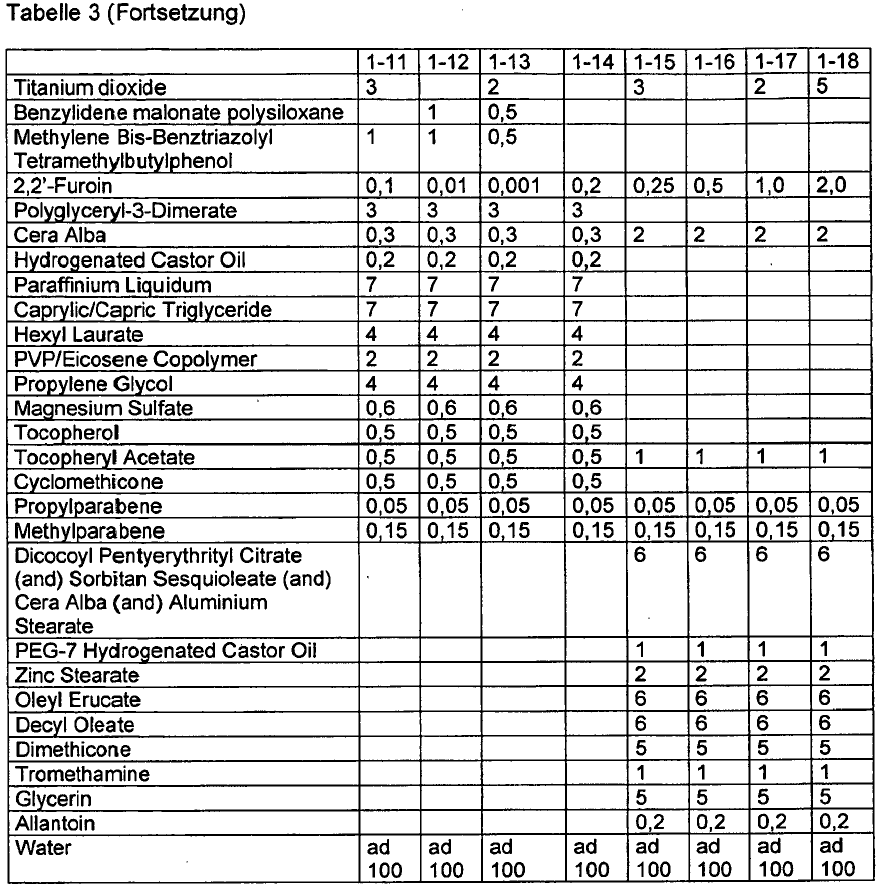

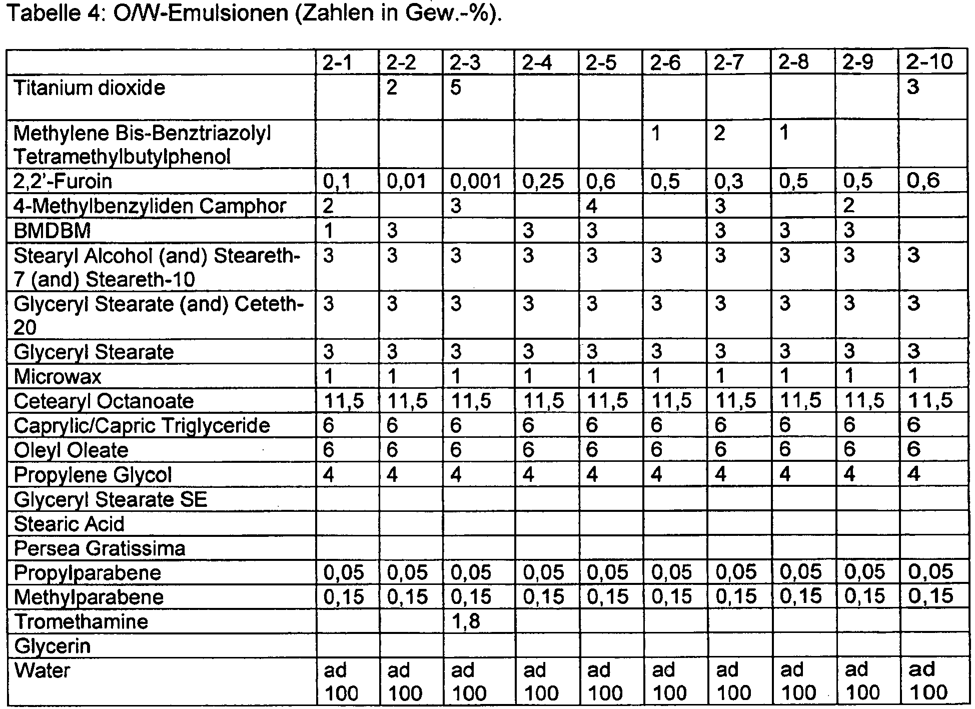

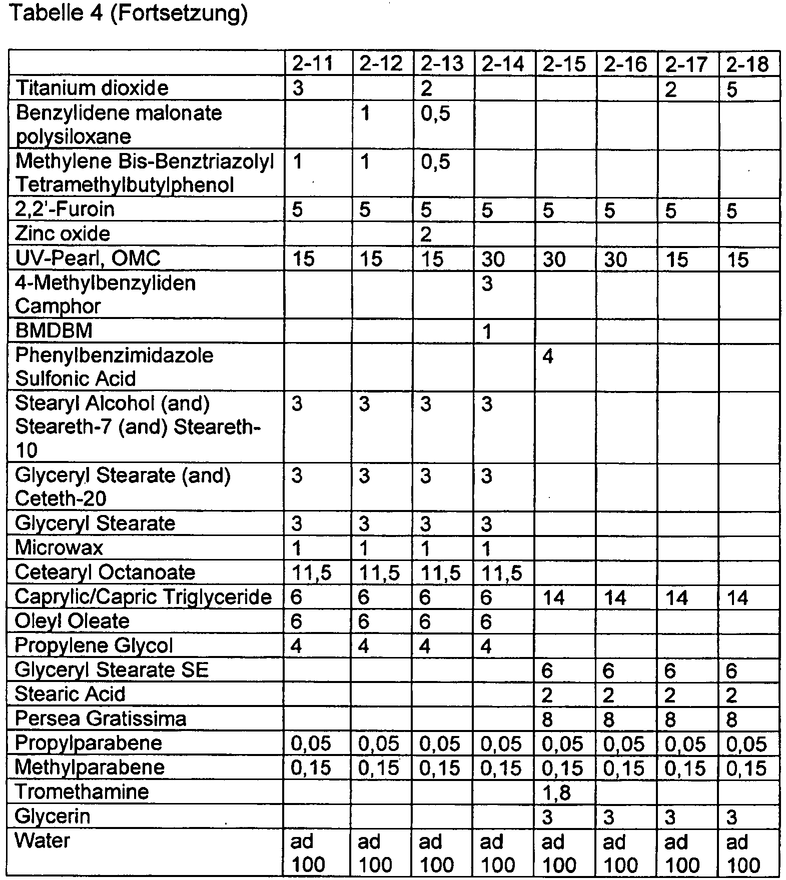

**Tabelle 5: Gele (Zahlen in Gew.-%).**

| | 3-1 | 3-2 | 3-3 | 3-4 | 3-5 | 3-6 | 3-7 | 3-8 | 3-9 | 3-10 |
|---|---|---|---|---|---|---|---|---|---|---|
| | | | | | | | | | | |
| Titanium dioxide | | 2 | 5 | | | | | | | 3 |
| 2,2'-Furoin | 0,4 | 0,5 | 0,01 | 0,7 | 0,4 | 0,3 | 0,001 | 0,7 | 0,6 | 0,7 |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl, Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Butylmethoxydibenzoylmethane | | 1 | | | | | | | | |
| Phenylbenzimidazole Sulfonic Acid | | | 4 | | | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0.5 | 0,5 | 0,5 |
| Caprylic/Capric Triplyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

## Patentansprüche

1. Verwendung von mindestens einer Verbindung der Formel (I) 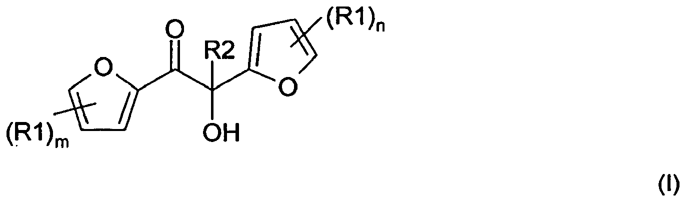 worin
R1 A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
R2 H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
Y H oder Alk,
A unverzweigtes oder verzweigtes Alkyl mit 1-18 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Alk unverzweigtes oder verzweigtes Alkyl mit 1-12 C-Atomen,
Cyc zyklisches Alkyl mit 3-10 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Ar einen ein-, zwei- oder dreikernigen aromatischen Carbozyklus mit 3 bis 20 C-Atomen, der unsubstituiert oder ein-, zwei- oder dreifach durch F, A, -(CY₂)ₚ-OY, -(CY₂)ₚ-NYY, -(CY₂)ₚ-COOY, -(CY₂)ₚ-CO-NYY oder -(CY₂)ₚ-NY-COY substituiert sein kann, und
m, n, p unabhängig voneinander 0, 1, 2 oder 3 bedeuten,
und/oder ihrer physiologisch unbedenklichen Salze, Tautomere, Stereoisomere und/oder Solvate, einschließlich deren Mischungen in allen Verhältnissen,
zur nicht-therapeutischen Aufhellung von Haut.

2. Verwendung von mindestens einer Verbindung der Formel (I) 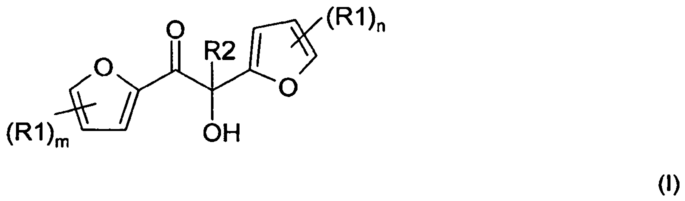 worin
R1 A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
R2 H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
Y H oder Alk,
A unverzweigtes oder verzweigtes Alkyl mit 1-18 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Alk unverzweigtes oder verzweigtes Alkyl mit 1-12 C-Atomen,
Cyc zyklisches Alkyl mit 3-10 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Ar einen ein-, zwei- oder dreikernigen aromatischen Carbozyklus mit 3 bis 20 C-Atomen, der unsubstituiert oder ein-, zwei- oder dreifach durch F, A, -(CY₂)ₚ-OY, -(CY₂)ₚ-NYY, -(CY₂)ₚ-COOY, -(CY₂)ₚ-CO-NYY oder -(CY₂)ₚ-NY-COY substituiert sein kann, und
m, n, p unabhängig voneinander 0, 1, 2 oder 3 bedeuten,
und/oder ihrer physiologisch unbedenklichen Salze, Tautomere, Stereoisomere und/oder Solvate, einschließlich deren Mischungen in allen Verhältnissen,
zur Hemmung von Tyrosinase in-vitro.

3. Verbindung der Formel (I) 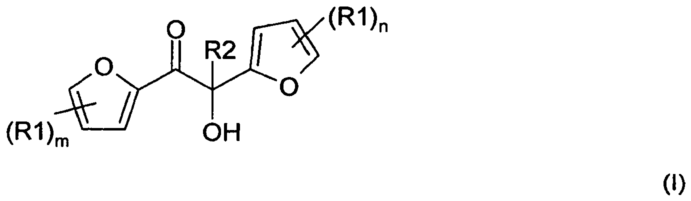 worin
R1 A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
R2 H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
Y H oder Alk,
A unverzweigtes oder verzweigtes Alkyl mit 1-18 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Alk unverzweigtes oder verzweigtes Alkyl mit 1-12 C-Atomen,
Cyc zyklisches Alkyl mit 3-10 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Ar einen ein-, zwei- oder dreikernigen aromatischen Carbozyklus mit 3 bis 20 C-Atomen, der unsubstituiert oder ein-, zwei- oder dreifach durch F, A, -(CY₂)ₚ-OY, -(CY₂)ₚ-NYY, -(CY₂)ₚ-COOY, -(CY₂)ₚ-CO-NYY oder -(CY₂)ₚ-NY-COY substituiert sein kann, und
m, n, p unabhängig voneinander 0, 1, 2 oder 3 bedeuten,
und/oder ihrer physiologisch unbedenklichen Salze, Tautomere, Stereoisomere und/oder Solvate, einschließlich deren Mischungen in allen Verhältnissen,
zur Verwendung in der Prophylaxe, Therapie und/oder Verlaufskontrolle von Pigmentstörungen der Haut.

4. Verbindung zur Verwendung nach Anspruch 3, wobei die Pigmentstörungen aus der Gruppe von Hyperpigmentierung, Sommersprossen, Altersflecken und Sonnenflecken ausgewählt sind.

5. Verwendung nach Anspruch 1 oder 2, wobei die mindestens eine Verbindung die Teilformel (IA) 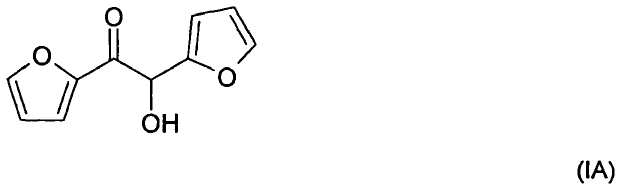 oder die Teilformel (IB) 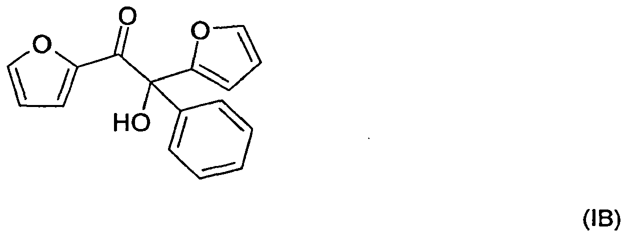 aufweist,
vorzugsweise die Teilformel (IA).

6. Verfahren zum Herrichten von Verbindungen der Formel (I) 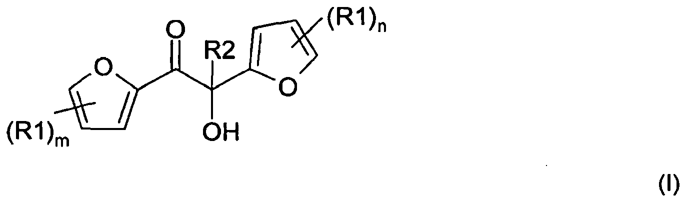 worin
R1 A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
R2 H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
Y H oder Alk,
A unverzweigtes oder verzweigtes Alkyl mit 1-18 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Alk unverzweigtes oder verzweigtes Alkyl mit 1-12 C-Atomen,
Cyc zyklisches Alkyl mit 3-10 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Ar einen ein-, zwei- oder dreikernigen aromatischen Carbozyklus mit 3 bis 20 C-Atomen, der unsubstituiert oder ein-, zwei- oder dreifach durch F, A, -(CY₂)ₚ-OY, -(CY₂)ₚ-NYY, -(CY₂)ₚ-COOY, -(CY₂)ₚ-CO-NYY oder -(CY₂)ₚ-NY-COY substituiert sein kann, und
m, n, p unabhängig voneinander 0, 1, 2 oder 3 bedeuten,
und/oder ihrer physiologisch unbedenklichen Salze, Tautomere, Stereoisomere und/oder Solvate, einschließlich deren Mischungen in allen Verhältnissen,
mit den folgenden Schritten:
(a) Umsetzen eines Furfural-Derivates der Formel (II) 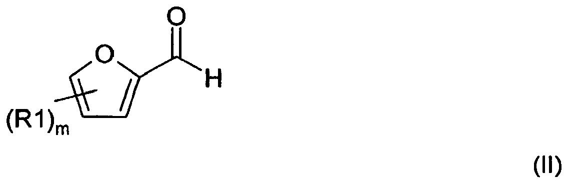 worin R1 und m die oben angegebene Bedeutung aufweisen,
mit einem Furfural-Derivat der Formel (III) 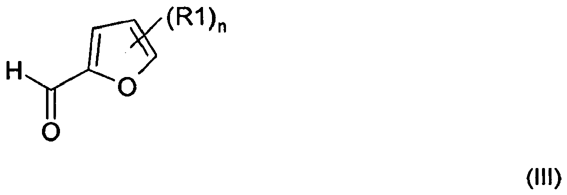 worin R1 und n die oben angegebene Bedeutung aufweisen,
in einer Benzoin-Addition unter Erhalt von Verbindungen der Formel (I) 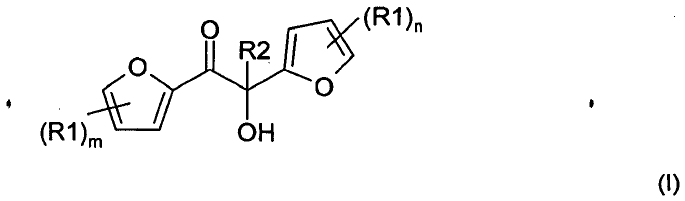 worin R1, R2, m und n die oben angegebene Bedeutung aufweisen,
und optional
(b) Umwandeln einer Base oder Säure der Verbindungen der Formel (I) in eines ihrer physiologisch unbedenklichen Salze,
und
(c) augenfälliges Herrichten der Verbindungen der Formel (I) oder eines ihrer physiologisch unbedenklichen Salze zur Verwendung nach einem der Ansprüche 1 bis 3.

7. Zubereitung enthaltend mindestens eine Verbindung der Formel (I) 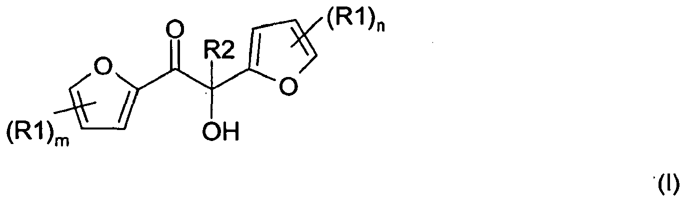 worin
R1 A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
R2 H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY oder Ar,
Y H oder Alk,
A unverzweigtes oder verzweigtes Alkyl mit 1-18 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Alk unverzweigtes oder verzweigtes Alkyl mit 1-12 C-Atomen,
Cyc zyklisches Alkyl mit 3-10 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- und/oder -C≡C- Gruppe ersetzt sein können,
Ar einen ein-, zwei- oder dreikernigen aromatischen Carbozyklus mit 3 bis 20 C-Atomen, der unsubstituiert oder ein-, zwei- oder dreifach dutch F, A, -(CY₂)ₚ-OY, -(CY₂)ₚ-NYY, -(CY₂)ₚ-COOY, -(CY₂)ₚ-CO-NYY oder -(CY₂)ₚ-NY-COY substituiert sein kann, und
m, n, p unabhängig voneinander 0, 1, 2 oder 3 bedeuten,
und/oder ihre physiologisch unbedenklichen Salze, Tautomere, Stereoisomere und/oder Solvate, einschließlich deren Mischungen in allen Verhältnissen,
in Kombination mit mindestens einem weiteren Wirkstoff, der aus der Gruppe von Antioxidantien, Vitaminen, UV-Filtern, hautaufhellenden Wirkstoffen, Selbstbräunungssubstanzen, antimikrobiellen Wirkstoffen, Hautfeuchteregulatoren, anti-inflammatorischen Wirkstoffen, Anti-Aging-Wirkstoffen, Anti-Cellulite-Wirkstoffen, Anti-Falten-Wirkstoffen und Anti-Schuppen-Wirkstoffen ausgewählt ist.

8. Zubereitung nach Anspruch 7, wobei ein oder mehrere Antioxidantien, ein oder mehrere Vitamine und/oder ein oder mehrere UV-Filter enthalten sind.

9. Zubereitung nach Anspruch 7 oder 8, wobei ein oder mehrere Selbstbräunungssubstanzen enthalten sind, vorzugsweise DHA, DHA plus, DHA rapid oder Erythrulose.

10. Zubereitung nach einem der Ansprüche 7 bis 9, wobei mindestens ein weiterer Wirkstoff oder Extrakt mit einer hautaufhellenden Aktivität enthalten ist, vorzugsweise ausgewählt aus der Gruppe von Hydrochinon, Niacinamid, Ascorbinsäure, physiologisch unbedenkliche Salze der Ascorbinsäure, Kojisäure, Arbutin, Aloesin, Azelainsäure, Elaginsäure und Rucinol oder Licorice-Extrakt, Maufbeerbaumextrakt und Emblica.

11. Zubereitung nach einem der Ansprüche 7 bis 10, wobei ein für kosmetische, pharmazeutische oder dermatologische Anwendungen geeigneter Träger und optional physiologisch unbedenkliche Hilfsstoffe und/oder Füllstoffe enthalten sind.

12. Verfahren zum Herstellen einer Zubereitung nach einem der Ansprüche 7 bis 11, wobei mindestens eine Verbindung der Formel (I) und/oder ihre physiologisch unbedenklichen Salze, Tautomere, Stereoisomere und/oder Solvate, einschließlich deren Mischungen in allen Verhältnissen, mit mindestens einem weiteren Wirkstoff und mindestens einem für topische Anwendungen geeigneten Träger und optional mit physiologisch unbedenklichen Hilfsstoffen und/oder Füllstoffen vermischt werden.

13. Verbindungen der Teilformel (I) 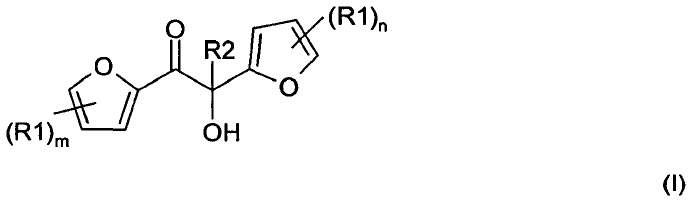 worin
R1 A, Cyc, OH oder Ar,
R2 Y, Cyc, OH oder Ar,
Y H oder Alk,
A unverzweigtes oder verzweigtes Alkyl mit 5-18 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- oder -C≡C- Gruppe ersetzt sein können,
Alk unverzweigtes oder verzweigtes Alkyl mit 1-12 C-Atomen,
Cyc zyklisches Alkyl mit 3-8 C-Atomen, wobei unabhängig voneinander 1-3 H-Atome durch F und/oder unabhängig voneinander eine oder zwei benachbarte CH₂-Gruppen durch -O-, -N(Alk)- oder eine -CH=CH- Gruppe ersetzt sein können,
Ar ein-, zwei- oder dreikerniges Aryl mit 5 bis 14 C-Atomen, das unsubstituiert oder ein-, zwei- oder dreifach durch F, Alk oder -(CY₂)ₚ-OY substituiert sein kann, und
m, p unabhängig voneinander 0, 1 oder 2 bedeuten,
unter der Maßgabe, dass Y und Ar für R2 ausgeschlossen sind, wenn m und n gleichzeitig 0 bedeuten,
und/oder ihre physiologisch unbedenklichen Salze, Tautomere, Stereoisomere und/oder Solvate, einschließlich deren Mischungen in allen Verhältnissen.

14. Verfahren zum Herstellen von Verbindungen der Teilformel (IC) 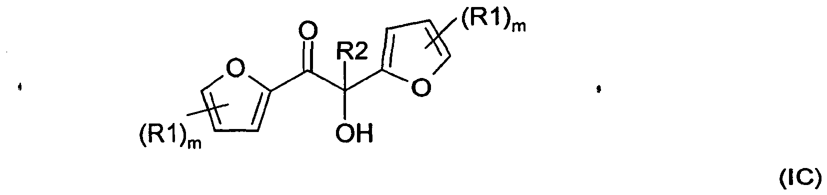 worin
R1 A, Cyc, OH oder Ar,
R2 Y, Cyc, OH oder Ar,
Y H oder Alk,
A unverzweigtes oder verzweigtes Alkyl mit 5-8 C-Atomen, wobei unabhängig voneinander 1-3 H-Atome durch F und/oder unabhängig voneinander eine oder zwei benachbarte CH₂-Gruppen durch -O-, -N(Alk)-, eine -CH=CH- oder -C≡C- Gruppe ersetzt sein können,
Alk unverzweigtes oder verzweigtes Alkyl mit 1-4 C-Atomen,
Cyc zyklisches Alkyl mit 3-8 C-Atomen, wobei unabhängig voneinander 1-3 H-Atome durch F und/oder unabhängig voneinander eine oder zwei benachbarte CH₂-Gruppen durch -O-, -N(Alk)- oder eine -CH=CH-Gruppe ersetzt sein können,
Ar ein-, zwei- oder dreikerniges Aryl mit 5 bis 14 C-Atomen, das unsubstituiert oder ein-, zwei- oder dreifach durch F, Alk oder -(CY₂)ₚ-OY substituiert sein kann, und
m, p unabhängig voneinander 0, 1 oder 2 bedeuten,
unter der Maßgabe, dass Y und Ar für R2 ausgeschlossen sind, wenn m 0 bedeutet,
und/oder ihre physiologisch unbedenklichen Salze, Tautomere, Stereoisomere und/oder Solvate, einschließlich deren Mischungen in allen Verhältnissen,
mit den folgenden Schritten:
(a) Benzoin-Addition von Furfural-Derivaten der Formel (II) 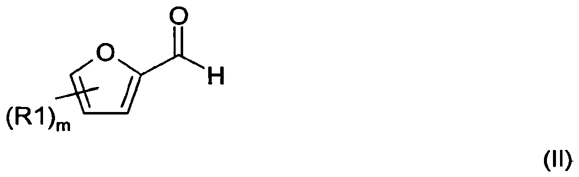 worin R1 und m die oben angegebene Bedeutung aufweisen,
unter Erhalt von Verbindungen der Teilformel (IC) 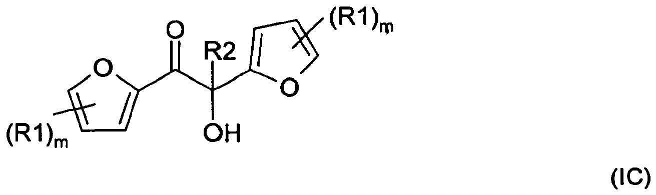 worin R1, R2 und m die oben angegebene Bedeutung aufweisen,
und optional
(b) Umwandeln einer Base oder Säure der Verbindungen der Teilformel (IC) in eines ihrer Salze.

15. Arzneimittel enthaltend mindestens eine Verbindung der Formel (I) nach Anspruch 13 und/oder ihre physiologisch unbedenklichen Salze, Tautomere, Stereoisomere und/oder Solvate, einschließlich deren Mischungen in allen Verhältnissen.

## Claims

1. Use of at least one compound of the formula (I) 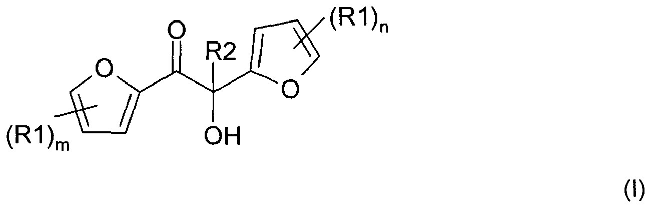 in which
R1 denotes A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
R2 denotes H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
Y denotes H or Alk,
A denotes unbranched or branched alkyl having 1-18 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Alk denotes unbranched or branched alkyl having 1-12 C atoms,
Cyc denotes cyclic alkyl having 3-10 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Ar denotes a mono-, bi- or tricyclic aromatic carbocycle having 3 to 20 C atoms, which may be unsubstituted or mono-, di- or trisubstituted by F, A, -(CY₂)ₚ-OY, -(CY₂)ₚ-NYY, -(CY₂)ₚ-COOY, -(CY₂)ₚ-CO-NYY or -(CY₂)ₚ-NY-COY, and
m, n, p, independently of one another, denote 0, 1, 2 or 3,
and/or physiologically acceptable salts, tautomers, stereoisomers and/or solvates thereof, including mixtures thereof in all ratios,
for the non-therapeutic lightening of skin.

2. Use of at least one compound of the formula (I) 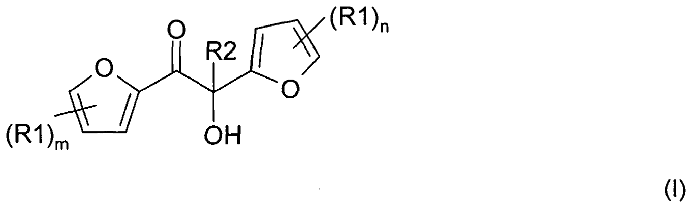 in which
R1 denotes A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
R2 denotes H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
Y denotes H or Alk,
A denotes unbranched or branched alkyl having 1-18 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Alk denotes unbranched or branched alkyl having 1-12 C atoms,
Cyc denotes cyclic alkyl having 3-10 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Ar denotes a mono-, bi- or tricyclic aromatic carbocycle having 3 to 20 C atoms, which may be unsubstituted or mono-, di- or trisubstituted by F, A, -(CY2)ₚ-OY, -(CY₂)ₚ-NYY, -(CY₂)ₚ-COOY, -(CY₂)ₚ-CO-NYY or -(CY₂)ₚ-NY-COY, and
m, n, p, independently of one another, denote 0, 1, 2 or 3,
and/or physiologically acceptable salts, tautomers, stereoisomers and/or solvates thereof, including mixtures thereof in all ratios,
for the inhibition of tyrosinase in vitro.

3. Compound of the formula (I) 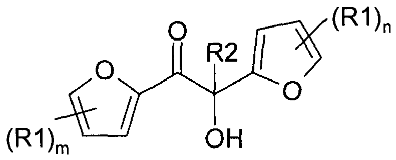 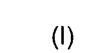 in which
R1 denotes A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
R2 denotes H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
Y denotes H or Alk,
A denotes unbranched or branched alkyl having 1-18 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Alk denotes unbranched or branched alkyl having 1-12 C atoms,
Cyc denotes cyclic alkyl having 3-10 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Ar denotes a mono-, bi- or tricyclic aromatic carbocycle having 3 to 20 C atoms, which may be unsubstituted or mono-, di- or trisubstituted by F, A, -(CY₂)ₚ-OY, -(CY₂)ₚ-NYY, -(CY₂)ₚ-COOY, -(CY₂)ₚ-CO-NYY or -(CY₂)ₚ-NY-COY, and
m, n, p, independently of one another, denote 0, 1, 2 or 3,
and/or physiologically acceptable salts, tautomers, stereoisomers and/or solvates thereof, including mixtures thereof in all ratios,
for use in the prophylaxis, therapy and/or progress control of pigment defects of the skin.

4. Compound for use according to Claim 3, where the pigment defects are selected from the group of hyperpigmentation, freckles, age spots and sun spots.

5. Use according to Claim 1 or 2, where the at least one compound has the part-formula (IA) 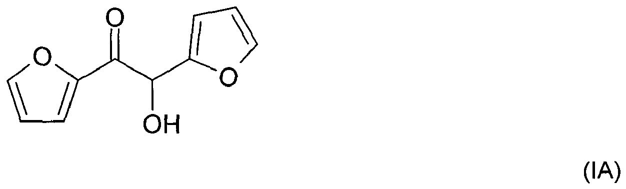 or the part-formula (IB) 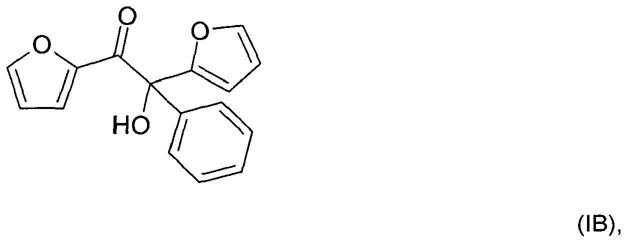 preferably the part-formula (IA).

6. Process for the preparation for administration of compounds of the formula (I) 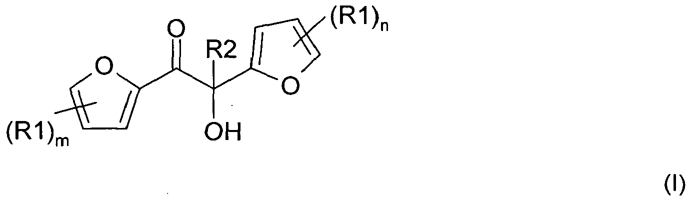 in which
R1 denotes A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
R2 denotes H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
Y denotes H or Alk,
A denotes unbranched or branched alkyl having 1-18 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Alk denotes unbranched or branched alkyl having 1-12 C atoms,
Cyc denotes cyclic alkyl having 3-10 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Ar denotes a mono-, bi- or tricyclic aromatic carbocycle having 3 to 20 C atoms, which may be unsubstituted or mono-, di- or trisubstituted by F, A, -(CY₂)ₚ-OY, -(CY₂)ₚ-NYY, -(CY₂)ₚ-COOY, -(CY₂)ₚ-CO-NYY or -(CY₂)ₚ-NY-COY, and
m, n, p, independently of one another, denote 0, 1, 2 or 3,
and/or physiologically acceptable salts, tautomers, stereoisomers and/or solvates thereof, including mixtures thereof in all ratios,
having the following steps:
(a) reaction of a furfural derivative of the formula (II) 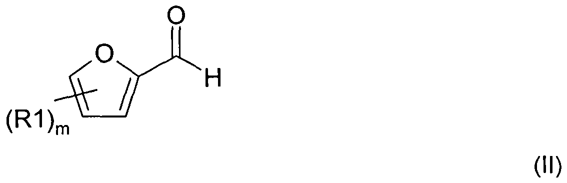 in which R1 and m have the meaning indicated above,
with a furfural derivative of the formula (III) 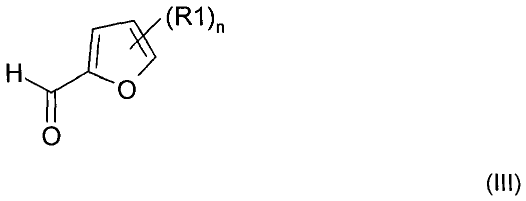 in which R1 and n have the meaning indicated above,
in a benzoin addition to give compounds of the formula (I) 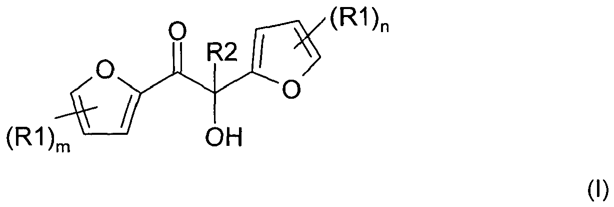 in which R1, R2, m and n have the meaning indicated above,
and optionally
(b) conversion of a base or acid of the compounds of the formula (I) into one of their physiologically acceptable salts,
and
(c) preparation for administration of the compounds of the formula (I) or one of their physiologically acceptable salts for the use according to one of Claims 1 to 3.

7. Preparation comprising at least one compound of the formula (I) 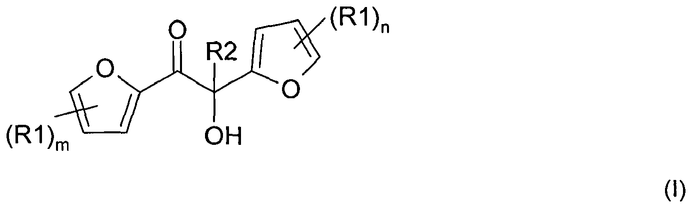 in which
R1 denotes A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
R2 denotes H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY or Ar,
Y denotes H or Alk,
A denotes unbranched or branched alkyl having 1-18 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Alk denotes unbranched or branched alkyl having 1-12 C atoms,
Cyc denotes cyclic alkyl having 3-10 C atoms, where, independently of one another, 1-3 H atoms may be replaced by F and/or, independently of one another, one or two adjacent CH₂ groups may be replaced by -O-, -N(Alk)-, a -CH=CH- and/or -C≡C- group,
Ar denotes a mono-, bi- or tricyclic aromatic carbocycle having 3 to 20 C atoms, which may be unsubstituted or mono-, di- or trisubstituted by F, A, -(CY₂)ₚ-OY, -(CY₂)ₚ-NYY, -(CY₂)ₚ-COOY, -(CY₂)ₚ-CO-NYY or -(CY₂)ₚ-NY-COY, and
m, n, p, independently of one another, denote 0, 1, 2 or 3,
and/or physiologically acceptable salts, tautomers, stereoisomers and/or solvates thereof, including mixtures thereof in all ratios,
in combination with at least one further active compound selected from the group of antioxidants, vitamins, UV filters, skin-lightening active compounds, self-tanning substances, antimicrobial active compounds, skin-moisture regulators, anti-inflammatory active compounds, anti-ageing active compounds, anticellulite active compounds, antiwrinkle active compounds and antidandruff active compounds.

8. Preparation according to Claim 7, where one or more antioxidants, one or more vitamins and/or one or more UV filters are present.

9. Preparation according to Claim 7 or 8, where one or more self-tanning substances are present, preferably DHA, DHA plus, DHA rapid or erythrulose.

10. Preparation according to one of Claims 7 to 9, where at least one further active compound or extract having a skin-lightening activity is present, preferably selected from the group of hydroquinone, niacinamide, ascorbic acid, physiologically acceptable salts of ascorbic acid, kojic acid, arbutin, aloesin, azelaic acid, ellagic acid and rucinol or liquorice extract, mulberry extract and emblica.

11. Preparation according to one of Claims 7 to 10, where a vehicle which is suitable for cosmetic, pharmaceutical or dermatological applications and optionally physiologically acceptable assistants and/or fillers are present.

12. Process for preparing a preparation according to one of Claims 7 to 11, where at least one compound of the formula (I) and/or physiologically acceptable salts, tautomers, stereoisomers and/or solvates thereof, including mixtures thereof in all ratios, are mixed with at least one further active compound and at least one vehicle which is suitable for topical applications and optionally with physiologically acceptable assistants and/or fillers.

13. Compounds of the part-formula (I) 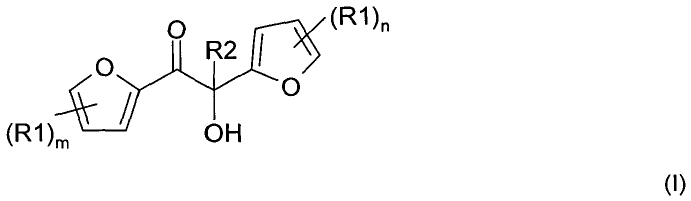 in which
R1 denotes A, Cyc, OH or Ar,
R2 denotes Y, Cyc, OH or Ar,
Y denotes H or Alk,
A denotes unbranched or branched alkyl having 5-18 C atoms, where, independently of one another, 1-3 H atoms may be replaced by F and/or, independently of one another, one or two adjacent CH₂ groups may be replaced by -O-, -N(Alk)-, a -CH=CH- or -C≡C- group,
Alk denotes unbranched or branched alkyl having 1-12 C atoms,
Cyc denotes cyclic alkyl having 3-8 C atoms, where, independently of one another, 1-3 H atoms may be replaced by F and/or, independently of one another, one or two adjacent CH₂ groups may be replaced by -O-, -N(Alk)- or a -CH=CH- group,
Ar denotes mono-, bi- or tricyclic aryl having 5 to 14 C atoms, which may be unsubstituted or mono-, di- or trisubstituted by F, Alk or -(CY₂)ₚ-OY, and
m, p, independently of one another, denote 0, 1 or 2,
with the proviso that Y and Ar are excluded for R2 if m and n simultaneously denote 0,
and/or physiologically acceptable salts, tautomers, stereoisomers and/or solvates thereof, including mixtures thereof in all ratios.

14. Process for the preparation of compounds of the part-formula (IC) 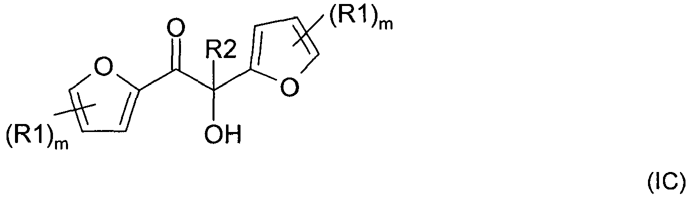 in which
R1 denotes A, Cyc, OH or Ar,
R2 denotes Y, Cyc, OH or Ar,
Y denotes H or Alk,
A denotes unbranched or branched alkyl having 5-8 C atoms, where, independently of one another, 1-3 H atoms may be replaced by F and/or, independently of one another, one or two adjacent CH₂ groups may be replaced by -O-, -N(Alk)-, a -CH=CH- or -C≡C- group,
Alk denotes unbranched or branched alkyl having 1-4 C atoms,
Cyc denotes cyclic alkyl having 3-8 C atoms, where, independently of one another, 1-3 H atoms may be replaced by F and/or, independently of one another, one or two adjacent CH₂ groups may be replaced by -O-, -N(Alk)- or a -CH=CH- group,
Ar denotes mono-, bi- or tricyclic aryl having 5 to 14 C atoms, which may be unsubstituted or mono-, di- or trisubstituted by F, Alk or -(CY₂)ₚ-OY, and
m, p, independently of one another, denote 0, 1 or 2,
with the proviso that Y and Ar are excluded for R2 if m denotes 0,
and/or physiologically acceptable salts, tautomers, stereoisomers and/or solvates thereof, including mixtures thereof in all ratios,
having the following steps:
(a) benzoin addition of furfural derivatives of the formula (II) 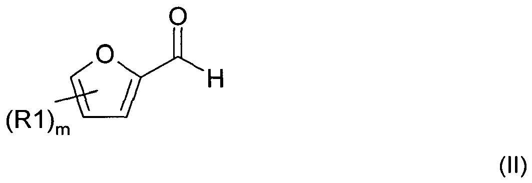 in which R1 and m have the meaning indicated above,
to give compounds of the part-formula (IC) 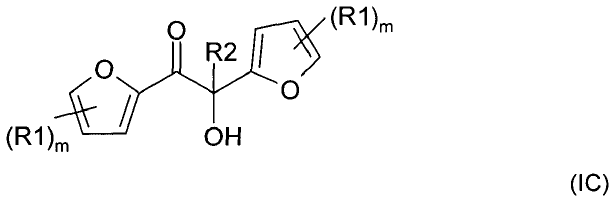 in which R1, R2 and m have the meaning indicated above,
and optionally
(b) conversion of a base or acid of the compounds of the part-formula (IC) into one of their salts.

15. Medicament comprising at least one compound of the formula (I) according to Claim 13 and/or physiologically acceptable salts, tautomers, stereoisomers and/or solvates thereof, including mixtures thereof in all ratios.

## Revendications

1. Utilisation d'au moins un composé de formule (I) 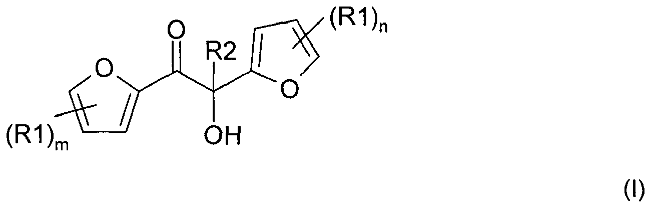 dans laquelle
R1 désigne A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
R2 désigne H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
Y désigne H ou Alk,
A désigne alkyle non ramifié ou ramifié ayant 1-18 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C=C-,
Alk désigne alkyle non ramifié ou ramifié ayant 1-12 atomes de C,
Cyc désigne alkyle cyclique ayant 3-10 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C≡C-,
Ar désigne un carbocycle mono-, bi- ou tricyclique aromatique ayant de 3 à 20 atomes de C, pouvant être non substitué ou mono-, di- ou trisubstitué par F, A, -(CY₂)ₚ-OY, -(CY₂)ₚ-NYY, -(CY₂)ₚ-COOY, -(CY₂)ₚ-CO-NYY ou -(CY₂)ₚ-NY-COY, et
m, n, p, indépendamment les uns des autres, désignent 0, 1, 2 ou 3,
et/ou les sels, tautomères, stéréoisomères et/ou solvats physiologiquement acceptables de celui-ci, y compris des mélanges de ceux-ci en toutes proportions,
pour l'éclaircissement non thérapeutique de la peau.

2. Utilisation d'au moins un composé de formule (I) 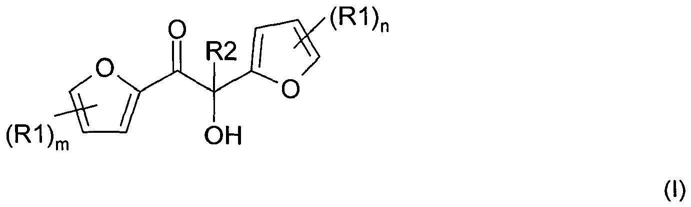 dans laquelle
R1 désigne A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
R2 désigne H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
Y désigne H ou Alk,
A désigne alkyle non ramifié ou ramifié ayant 1-18 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C≡C-,
Alk désigne alkyle non ramifié ou ramifié ayant 1-12 atomes de C,
Cyc désigne alkyle cyclique ayant 3-10 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C≡C-,
Ar désigne un carbocycle mono-, bi- ou tricyclique aromatique ayant de 3 à 20 atomes de C, pouvant être non substitué ou mono-, di- ou trisubstitué par F, A, -(CY₂)ₚ-OY, -(CY₂)ₚ-NYY, -(CY₂)ₚ-COOY, -(CY₂)ₚ-CO-NYY ou -(CY₂)ₚ-NY-COY, et
m, n, p, indépendamment les uns des autres, désignent 0, 1, 2 ou 3,
et/ou les sels, tautomères, stéréoisomères et/ou solvats physiologiquement acceptables de celui-ci, y compris des mélanges de ceux-ci en toutes proportions, pour l'inhibition de tyrosinase *in vitro*

3. Composé de formule (I) 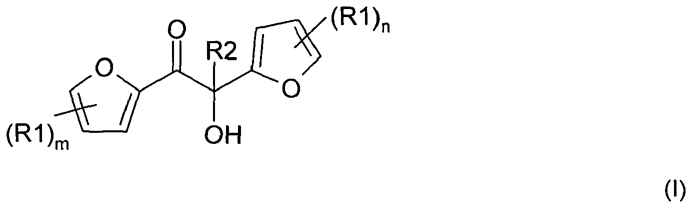 dans laquelle
R1 désigne A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
R2 désigne H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
Y désigne H ou Alk,
A désigne alkyle non ramifié ou ramifié ayant 1-18 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C≡C-,
Alk désigne alkyle non ramifié ou ramifié ayant 1-12 atomes de C,
Cyc désigne alkyle cyclique ayant 3-10 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C≡C-,
Ar désigne un carbocycle mono-, bi- ou tricyclique aromatique ayant de 3 à 20 atomes de C, pouvant être non substitué ou mono-, di- ou trisubstitué par F, A, -(CY₂)ₚ-OY, -(CY₂)ₚ-NYY, -(CY₂)ₚ-COOY, -(CY₂)ₚ-CO-NYY ou -(CY₂)ₚ-NY-COY, et
m, n, p, indépendamment les uns des autres, désignent 0, 1, 2 ou 3,
et/ou les sels, tautomères, stéréoisomères et/ou solvats physiologiquement acceptables de celui-ci, y compris des mélanges de ceux-ci en toutes proportions, pour une utilisation dans la prophylaxie, la thérapie et/ou le contrôle de la progression de défauts de pigmentation de la peau.

4. Composé pour une utilisation selon la revendication 3, **caractérisé en ce que** les défauts de pigmentation sont choisis dans le groupe constitué par l'hyperpigmentation, les taches de rousseur, le lentigo sénile et le lentigo solaire.

5. Utilisation selon la revendication 1 ou 2, **caractérisée en ce que** le au moins un composé possède la formule partielle (IA) 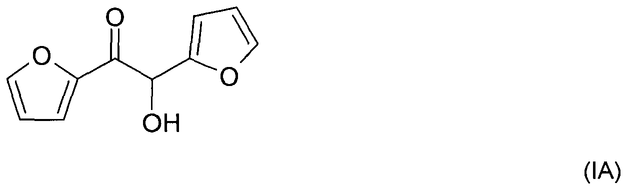 ou la formule partielle (IB) 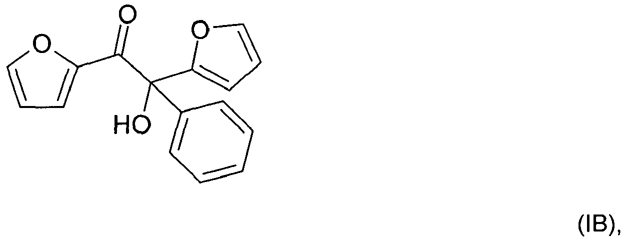 préférablement la formule partielle (IA).

6. Procédé de préparation pour l'administration de composés de formule (I) 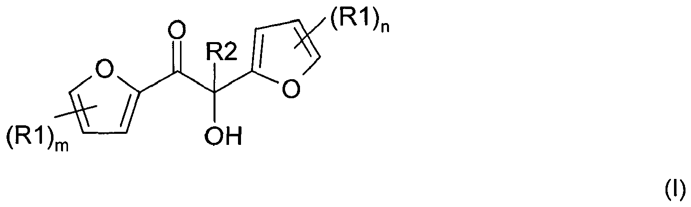 dans laquelle
R1 désigne A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
R2 désigne H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
Y désigne H ou Alk,
A désigne alkyle non ramifié ou ramifié ayant 1-18 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C≡C-,
Alk désigne alkyle non ramifié ou ramifié ayant 1-12 atomes de C,
Cyc désigne alkyle cyclique ayant 3-10 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C≡C-,
Ar désigne un carbocycle mono-, bi- ou tricyclique aromatique ayant de 3 à 20 atomes de C, pouvant être non substitué ou mono-, di- ou trisubstitué par F, A, -(CY₂)ₚ-OY, -(CY₂)ₚ-NYY, -(CY₂)ₚ-COOY, -(CY₂)ₚ-CO-NYY ou -(CY₂)ₚ-NY-COY, et
m, n, p, indépendamment les uns des autres, désignent 0, 1, 2 ou 3,
et/ou les sels, tautomères, stéréoisomères et/ou solvats physiologiquement acceptables de ceux-ci, y compris des mélanges de ceux-ci en toutes proportions,
comportant les étapes suivantes :
(a) la réaction d'un dérivé de furfural de formule (II) 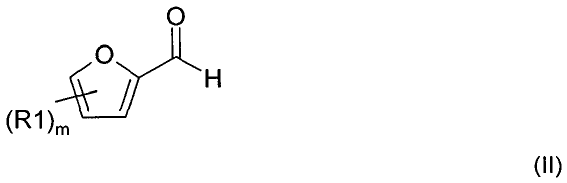 dans laquelle R1 et m revêtent la signification indiquée ci-dessus,
avec un dérivé de furfural de formule (III) 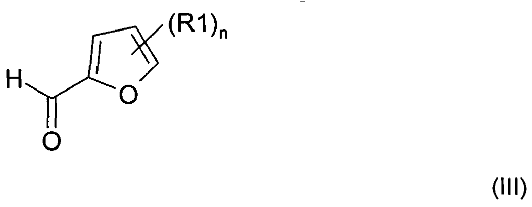 dans laquelle R1 et n revêtent la signification indiquée ci-dessus,
dans une addition de benzoïne, pour donner les composés de formule (I) 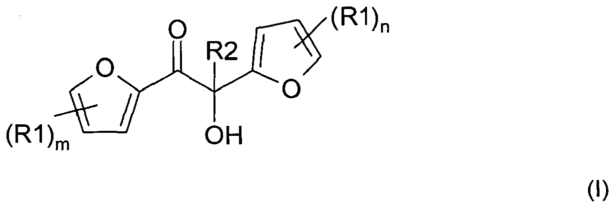 dans laquelle R1, R2, m et n revêtent la signification indiquée ci-dessus,
et éventuellement
(b) la conversion d'une base ou d'un acide des composés de formule (I) en l'un de leurs sels physiologiquement acceptables,
et
(c) la préparation pour l'administration des composés de formule (I) ou de l'un de leurs sels physiologiquement acceptables pour l'utilisation selon l'une des revendications 1 à 3.

7. Préparation comprenant au moins un composé de formule (I) 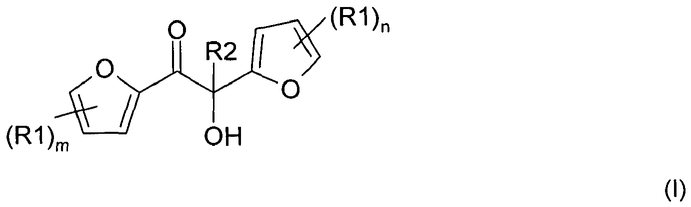 dans laquelle
R1 désigne A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
R2 désigne H, A, Cyc, F, -(CY₂)ₚ-OY, -(CY₂)ₚ-NYY, -(CY₂)ₚ-COOY, -(CY₂)ₚ-CO-NYY, -(CY₂)ₚ-NY-COY ou Ar,
Y désigne H ou Alk,
A désigne alkyle non ramifié ou ramifié ayant 1-18 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C≡C-,
Alk désigne alkyle non ramifié ou ramifié ayant 1-12 atomes de C,
Cyc désigne alkyle cyclique ayant 3-10 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- et/ou -C≡C-,
Ar désigne un carbocycle mono-, bi- ou tricyclique aromatique ayant de 3 à 20 atomes de C, pouvant être non substitué ou mono-, di- ou trisubstitué par F, A, -(CY₂)ₚ-OY, -(CY₂)ₚ-NYY, -(CY₂)ₚ-COOY, -(CY₂)ₚ-CO-NYY ou -(CY₂)ₚ-NY-COY, et
m, n, p, indépendamment les uns des autres, désignent 0, 1, 2 ou 3,
et/ou les sels, tautomères, stéréoisomères et/ou solvats physiologiquement acceptables de celui-ci, y compris des mélanges de ceux-ci en toutes proportions,
en combinaison avec au moins un autre composé actif choisi dans le groupe constitué par les antioxydants, les vitamines, les filtres anti-UV, les composés actifs d'éclaircissement de la peau, les substances auto-bronzantes, les composés actifs antimicrobiens, les régulateurs de l'humidité de la peau, les composés actifs anti-inflammatoires, les composés actifs antivieillissement, les composés actifs anticellulite, les composés actifs antirides et les composés actifs antipelliculaires.

8. Préparation selon la revendication 7, **caractérisée en ce qu'**un ou plusieurs antioxydants, une ou plusieurs vitamines et/ou un ou plusieurs filtres anti-UV, sont présents.

9. Préparation selon la revendication 7 ou 8, **caractérisée en ce qu'**une ou plusieurs substances auto-bronzantes sont présentes, préférablement le DHA, le DHA plus, le DHA rapide ou l'érythrulose.

10. Préparation selon l'une des revendications 7 à 9, **caractérisée en ce qu'**au moins un autre composé actif ou extrait ayant une activité d'éclaircissement de la peau est présent, choisi de préférence dans le groupe constitué par l'hydroquinone, le niacinamide, l'acide ascorbique, les sels physiologiquement acceptables de l'acide ascorbique, l'acide kojique, l'arbutine, l'aloésine, l'acide azélaïque, l'acide ellagique et le rucinol ou l'extrait de réglisse, l'extrait de mûre et l'emblica.

11. Préparation selon l'une des revendications 7 à 10, **caractérisée en ce qu'**un véhicule qui est convenable pour des applications cosmétiques, pharmaceutiques ou dermatologiques et éventuellement des auxiliaires et/ou des charges physiologiquement acceptables, sont présents.

12. Procédé de préparation d'une préparation selon l'une des revendications 7 à 11, **caractérisé en ce qu'**au moins un composé de formule (I) et/ou des sels, tautomères, stéréoisomères et/ou solvats physiologiquement acceptables de celui-ci, y compris des mélanges de ceux-ci en toutes proportions, sont mélangés avec au moins un autre composé actif et au moins un véhicule qui est convenable pour des applications topiques et éventuellement avec des auxiliaires et/ou des charges physiologiquement acceptables.

13. Composés de formule partielle (I) 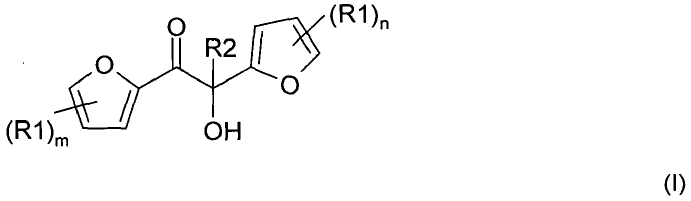 dans laquelle
R1 désigne A, Cyc, OH ou Ar,
R2 désigne Y, Cyc, OH ou Ar,
Y désigne H ou Alk,
A désigne alkyle non ramifié ou ramifié ayant 5-18 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- ou -C=C-,
Alk désigne alkyle non ramifié ou ramifié ayant 1-12 atomes de C,
Cyc désigne alkyle cyclique ayant 3-8 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)- ou un groupement -CH=CH-,
Ar désigne aryle mono-, bi- ou tricyclique ayant de 5 à 14 atomes de C, pouvant être non substitué ou mono-, di- ou trisubstitué par F, Alk ou -(CY₂)ₚ-OY, et
m, p, indépendamment les uns des autres, désignent 0, 1 ou 2,
à condition que Y et Ar soient exclus pour R2 si m et n désignent simultanément 0, et/ou des sels, tautomères, stéréoisomères et/ou solvats physiologiquement acceptables de ceux-ci, y compris des mélanges de ceux-ci en toutes proportions.

14. Procédé de préparation de composés de formule partielle (IC) 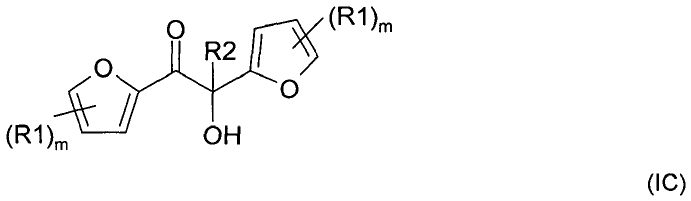 dans laquelle
R1 désigne A, Cyc, OH ou Ar,
R2 désigne Y, Cyc, OH ou Ar,
Y désigne H ou Alk,
A désigne alkyle non ramifié ou ramifié ayant 5-8 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)-, un groupement -CH=CH- ou -C≡C-,
Alk désigne alkyle non ramifié ou ramifié ayant 1-4 atomes de C,
Cyc désigne alkyle cyclique ayant 3-8 atomes de C, où, indépendamment les uns des autres, 1-3 atomes de H peuvent être remplacés par F et/ou, indépendamment les uns des autres, un ou deux groupements CH₂ adjacents peuvent être remplacés par -O-, -N(Alk)- ou un groupement -CH=CH-,
Ar désigne aryle mono-, bi- ou tricyclique ayant de 5 à 14 atomes de C, pouvant être non substitué ou mono-, di- ou trisubstitué par F, Alk ou -(CY₂)ₚ-OY, et
m, p, indépendamment les uns des autres, désignent 0, 1 ou 2,
à condition que Y et Ar soient exclus pour R2 si m désigne 0,
et/ou les sels, tautomères, stéréoisomères et/ou solvats physiologiquement acceptables de ceux-ci, y compris des mélanges de ceux-ci en toutes proportions,
comportant les étapes suivantes
(a) l'addition de benzoïne de dérivés de furfural de formule (II) 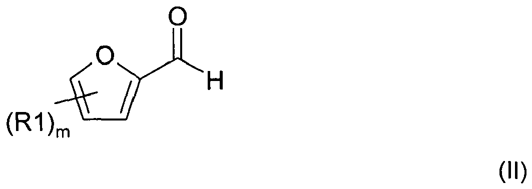 dans laquelle R1 et m revêtent la signification indiquée ci-dessus,
pour donner les composés de formule partielle (IC) 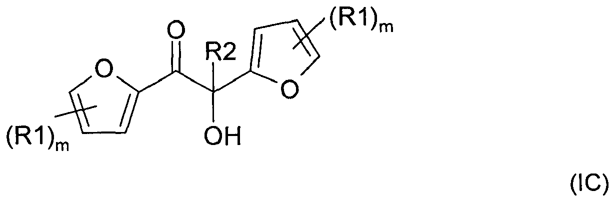 dans laquelle R1, R2 et m revêtent la signification indiquée ci-dessus,
et éventuellement
(b) la conversion d'une base ou d'un acide des composés de formule partielle (IC) en l'un de leurs sels.

15. Médicament comprenant au moins un composé de formule (I) selon la revendication 13 et/ou des sels, tautomères, stéréoisomères et/ou solvats physiologiquement acceptables de celui-ci, y compris des mélanges de ceux-ci en toutes proportions.
